# EUROPEAN PATENT SPECIFICATION

(11) **EP 1 572 929 B1**
(45) Date of publication and mention of the grant of the patent: **11.05.2011**
(21) Application number: 03730447.4
(22) Date of filing: 12.06.2003
(51) Int. Cl.: C07H 21/02

(54) **MEMBRANE-ANCHORED BETA2 MICROGLOBULIN COVALENTLY LINKED TO MHC CLASS I PEPTIDE EPITOPES**
"MIT MHC-KLASSE-I-PEPTIDEPITOPEN KOVALENT VERKN PFTES MEMBRANVERANKERTES BETA2-MIKROGLOBULIN"
BETA2 MICROGLOBULINE ANCREE SUR MEMBRANE LIEE DE FACON COVALENTE A DES EPITOPES PEPTIDIQUES DU CMH DE CLASSE I

(30) Priority: 12.06.2002 US 388273 P
(43) Date of publication of application: 14.09.2005
(73) Proprietor: GAVISH-GALILEE BIO APPLICATIONS LTD, 46905 Kibbutz Glil Yam (IL)
(72) Inventor: GROSS, Gideon, 12 340 M.P. Korazim (IL); MARGALIT, Alon, 25180 M.P. Western Galilee (IL)
(74) Representative: Vossius & Partner
(86) International application number: PCT/IL2003/000501
(87) International publication number: WO 2003/106616

(56) References cited:
- WO-A-01/91698
- WO-A-99/02183
- WO-A2-01/72768
- US-A1- 2004 086 960
- YU YIK Y L ET AL: "Cutting edge: single-chain trimers of MHC class I molecules form stable structures that potently stimulate antigen-specific T cells and B cells." JOURNAL OF IMMUNOLOGY (BALTIMORE, MD. : 1950) 1 APR 2002, vol. 168, no. 7, 1 April 2002 (2002-04-01), pages 3145-3149, XP002433697 ISSN: 0022-1767
- VAN DEN EYNDE B J ET AL: "T cell defined tumor antigens" CURRENT OPINION IN IMMUNOLOGY, CURRENT BIOLOGY LTD, vol. 9, no. 5, October 1997 (1997-10), pages 684-693, XP004313587 ISSN: 0952-7915
- TAFURO S ET AL: "RECONSTITUTION OF ANTIGEN PRESENTATION IN HLA CLASS I-NEGATIVE CANCER CELLS WITH PEPTIDE-BETA2M FUSION MOLECULES" EUROPEAN JOURNAL OF IMMUNOLOGY, WEINHEIM, DE, vol. 31, no. 2, February 2001 (2001-02), pages 440-449, XP001020497 ISSN: 0014-2980
- WEN Y J ET AL: "Immunogenicity and cross-reactivity with idiotypic IgA of VH CDR3 peptide in multiple myeloma." BRITISH JOURNAL OF HAEMATOLOGY MAR 1998, vol. 100, no. 3, March 1998 (1998-03), pages 464-468, XP002433771 ISSN: 0007-1048
- NAYAK J.V. ET AL: 'Antigen delivery to dendritic cells: Implications for cancer immunotherapy' CLINICAL IMMUNOLOGY NEWSLETTER 199910 US LNKD- DOI:10.1016/S0197-1859(00)87087-9 vol. 19, no. 10-11, October 1999, pages 131 - 135 ISSN: 0197-1859
- TOSHITANI K. ET AL: 'EXPRESSION OF A SINGLE-CHAIN HLA CLASS I MOLECULE IN A HUMAN CELL LINE: PRESENTATION OF EXOGENOUS PEPTIDE AND PROCESSED ANTIGEN TO CYTOTOXIC T LYMPHOCYTES' PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.93.1.236 vol. 93, no. 1, 09 January 1996, pages 236 - 240, XP002051157 ISSN: 0027-8424
- UGER R.A.; CHAN S.M.; BARBER B.H.: 'Covalent linkage to beta 2-microglobulin enhances the MHC stability and antigenicity of suboptimal CTL epitopes' JOURNAL OF IMMUNOLOGY, AMERICAN ASSOCIATION OF IMMUNOLOGISTS, US vol. 162, no. 10, 01 January 1999, pages 6024 - 6028, XP002959840 ISSN: 0022-1767
- NAIR S.K. ET AL: 'ANTIGEN-PRESENTING CELLS PULSED WITH UNFRACTIONATED TUMOR-DERIVED PEPTIDES ARE POTENT TUMOR VACCINES' EUROPEAN JOURNAL OF IMMUNOLOGY, WILEY - V C H VERLAG GMBH & CO. KGAA, DE LNKD- DOI:10.1002/EJI.1830270304 vol. 27, no. 3, 01 January 1997, pages 589 - 597, XP001096188 ISSN: 0014-2980
- BERKO DIKLA ET AL: "Membrane-anchored beta2-microglobtilin stabilizes a highly receptive state of MHC class I molecules" JOURNAL OF IMMUNOLOGY, vol. 174, no. 4, 15 February 2005 (2005-02-15), pages 2116-2123, ISSN: 0022-1767
- MARGALIT A. ET AL: 'Chimeric beta2 microglobulin/CD3zeta polypeptides expressed in T cells convert MHC class I peptide ligands into T cell activation receptors: A potential tool for specific targeting of pathogenic CD8+ T cells' INTERNATIONAL IMMUNOLOGY, OXFORD UNIVERSITY PRESS, GB LNKD- DOI:10.1093/INTIMM/DXG136 vol. 15, no. 11, 01 November 2003, pages 1379 - 1387, XP009039662 ISSN: 0953-8178
- BJORKMAN P.J. ET AL: 'STRUCTURE OF THE HUMAN CLASS I HISTOCOMPATIBILITY ANTIGEN HLA-A2' NATURE (LONDON) vol. 329, no. 6139, 1987, pages 506 - 512 ISSN: 0028-0836

## Description

### FIELD OF THE INVENTION

The present invention is in the field of Immunology and relates to DNA molecules encoding chimeric polypeptides comprising β₂-microglobulin and a polypeptide stretch for anchoring the β₂-microglobulin molecule to the cell membrane, herein referred to as single-chimeric β₂-microglobulin (scβ₂m), and to such DNA molecules further comprising at least one antigenic peptide linked to the amino terminal of the β₂-microglobulin molecule, herein referred to as double-chimeric β₂-microglobulin (dcβ₂m), wherein the antigenic peptide is tumor-associated antigen (TAA) , and to antigen-presenting cells expressing said scβ₂m and dcβ₂m polypeptides, as novel tools for efficient CTL induction for the treatment of cancer and infectious diseases.

**ABBREVIATIONS: APC:** antigen-presenting cell; β₂**m:** β₂-microglobulin; **BCR:** B cell receptor; **CDR:** complementarity-determining region; **CTL:** cytotoxic T lymphocyte; **dc**β**₂m:** double-chimeric β₂-microglobulin; **DC:** dendritic cells; **ER:** endoplasmic reticulum; **GPI:** glycosyl-phosphatidylinositol; **Ha:** hemagglutinin; **h**β**₂m:** human β₂-microglobulin; **HLA:** human leukocyte antigen (=human MHC); **Ig:** immunoglobulin; **ITAM:** immunoreceptor tyrosine-based activation motif; **mAb:** monoclonal antibody; **m**β**₂m:** mouse β₂-microglobulin; **MFI:** mean fluorescence intensity; **MHC:** major histocompatibility complex; **NP:** nucleoprotein; **OVA:** chicken ovalbumin; **RT-PCR:** reverse transcriptase-polymerase chain reaction; **sc**β**₂m:** single-chimeric β₂-microglobulin **TAA:** tumor associated antigen; **TAP:** transporter associated with antigen processing; **TCR:** T-cell receptor; **T**_{H} **:** T helper cells; **TRP:** tyrosinase-related protein.

### BACKGROUND OF THE INVENTION

The discovery, in recent years, of tumor associated antigens (TAAs) in a growing list of primary human tumors has led to the recognition that most, if not all types of human cancers express tumor antigens. The realization that some TAAs can elicit immune responses that lead to tumor rejection, has refueled interest in the field of cancer immunology, raising hopes for the development of potent anticancer immunotherapeutic tools and cancer vaccines (for reviews, see Minev et al., 1999; Gilboa et al., 1998; Rosenberg, 1999).

Tumor antigens can be divided according to the type of immune response they induce: humoral or cellular, which can be further subdivided into CD4⁺ (helper) and CD8⁺ (cytotoxic) T cell responses. Most TAAs known today were identified by their ability to induce cellular responses, predominantly by cytotoxic T lymphocytes (CTLs). CTLs utilize their clonotypic T cell receptor (TCR) to recognize antigenic peptides presented on major histocompatibility complex (MHC) class I molecules, which are expressed by most nucleated cells in the body. These proteins consist of a membrane-attached α heavy chain, which harbors three structurally distinct extracellular domains (α1-α3), and a non-covalently associated β₂ microglobulin (β₂m) light chain, that is not anchored to the cell membrane. Peptides, typically 8-10 amino acids long, bind to a special groove formed between the two membrane-distal domains of the α chain, α1 and α2, mainly via 2-3 dominant anchor residues.

CTLs serve as the major effector arm of the immune system and represent an important component of an animal's or an individual's immune response against a variety of pathogens and cancers. CTLs which have been specifically activated against a particular antigen are capable of killing the cell that contains or expresses the antigen. CTLs are particularly important in providing an effective immune response against intracellular pathogens, such as a wide variety of viruses, and some bacteria and parasites, as well as against tumors.

Some tumors down-regulate MHC class I expression, implying a strong selective pressure imposed by CTLs. In addition, CTLs are capable of recognizing single amino acid substitutions such as those that occur in TAAs resulting from point mutations. All these suggest that TAAs-derived MHC class I peptides are likely to constitute effective rejection antigens.

CTL activation, or priming, requires that antigenic peptides be presented initially on professional antigen-presenting cells (APCs), primarily dendritic cells (DCs), in secondary lymphoid organs (Steinman, 1989). In addition to highly efficient antigen presentation, DCs provide a co-stimulatory signal, which is mandatory for T cell priming, usually by engagement of their up-regulated B7 molecules with their CD28 receptor on the T cell (Janeway and Bottomly, 1994). Acquisition of the ability of the DC to prime CTLs is primarily mediated by antigen-specific CD4 T cells in a process referred to as 'licensing'. It involves interaction of the TCR of the CD4 T cell with an antigenic peptide on an MHC class II molecule on the DC and concomitant engagement of the CD40 ligand (CD40L) on the T cell with the DC CD40 receptor (Guermonprez et al., 2002). Another unique feature of DCs is their ability to present peptides generated from exogenous proteins on their MHC class I molecules, a phenomenon generally referred to as cross-presentation (Heath and Carbone, 2001). Indeed, it is due to these unique properties, that autologous DCs are considered ideal for the induction of antitumor responses (for reviews, see Gilboa et al., 1998; Nouri-Shirazi et al., 2000; Chen et al., 2000; Porgador et al., 1996) and are thus widely explored as potential cancer vaccines.

Attempts to develop novel approaches for the generation of cancer vaccines have taken two major routes. Some approaches make use of the complete antigenic repertoire of the tumor cells. This is accomplished by induction of T cells by irradiated tumor cells, genetically modified to express cytokines, co-stimulatory molecules or foreign MHC, by pulsing of DCs with tumor-derived heat shock proteins, whole tumor cell extracts or total RNA (a minute amount of which can easily be amplified) and fusion of DCs with tumor cells (Zhang et al., 1997; Gong et al., 1997; Gong et al., 2000). These strategies are applicable to many types of tumors and, in theory, can induce a wide spectrum of antitumor CTLs. However, presentation of TAA-derived peptides of potential clinical benefit is not enriched and these protocols may thus fail to induce therapeutic CTLs (Sogn, 2000; Dalgleish, 2001). Furthermore, these procedures do not allow attribution of clinical response to particular antigens and, therefore, useful information cannot be deduced for broader implementation.

Other approaches for the generation of cancer vaccines are based on known TAAs. These include the design of peptide, DNA and recombinant viral vaccines, charging DCs with either purified tumor-associated proteins or TAA-derived peptides and presentation of TAA-derived peptides, which are produced following gene delivery into autologous or syngeneic (in mice) DCs (for review, see Gilboa et al., 1998).

Indeed, some encouraging data showing CTL induction and vaccine efficacy came from animal studies exploring either type of above-described approaches. However, clinical success in human trials has so far been limited, with little correlation between the observed number of specific anti-tumor CTLs and the actual clinical response (Sogn, 1998; Moingeon, 2001; Jager et al., 2002). This is attributed, in part, to requirement for help from CD4⁺ cells and to immunosuppressing cytokines produced by the tumor cells, but also to the fact that many of the identified MHC class I-associated TAA peptides are poorly presented on the cell surface because of low level of protein expression and low affinity for their restricting MHC class I molecule (Watson et al., 1995; Vora et al., 1997).

Intracellular proteins, as well as soluble protein antigens delivered into the cytoplasm of a cell, are degraded into short peptides by a cytosolic proteolytic system present in all cells. Those proteins targeted for proteolysis often have a small protein, called ubiquitin, attached covalently to a lysine-amino group near the amino terminal of the protein. These ubiquitin-protein complexes are degraded into a variety of peptides by a multifunctional protease complex called proteasome. Experimental evidence indicates that the immune system utilizes this general pathway of protein degradation to produce small peptides for presentation with class I MHC molecules. The peptides, generated in the cytosol by the proteasome, are translocated by a transporter protein, called TAP (for "transporter associated with antigen processing"), into the endoplasmic reticulum (ER), by a process that requires the hydrolysis of ATP. Within the ER membrane, newly synthesized class I α chain associates with calnexin until β₂m binds to the α chain. The class I α chain-β₂m heterodimer then binds to calreticulin and the TAP-associated protein tapasin. When a peptide delivered by TAP is bound to the class I molecule, folding of MHC class I is complete and it is released from the ER and transported to the surface of the cell. TAP has the highest affinity for peptides containing 8-13 amino acids. Peptides longer than the size required for MHC class I binding are further trimmed in the ER by assigned amino peptidases to acquire the optimal length.

A single cell can display thousands of different MHC class I bound peptides, most of them only at low frequency of less than 0.1% of the total. The density of MHC/peptide complexes on the cell surface determines the degree of T cell responsiveness (Levitsky et al., 1996; Tsomides et al., 1994; Gervois et al., 1996). CTL priming by a professional APC generally requires a higher density of specific complexes than that required on the surface of the target cell for activation of an armed effector CTL (Armstrong et al., 1998; Reis e Souza, 2001). The ability to generate high numbers of particular MHC class I/peptide complexes on the APC itself could, therefore, be of great value for elicitation of strong CTL responses, which may be effective against TAA-derived peptides of an otherwise limited distribution.

This realization has prompted attempts to enhance level of peptide presentation by APCs, either by increasing the intrinsic affinity of the peptide for the restricting MHC class I molecule, or by manipulations aiming at elevating the actual number of specific classI/peptide complexes on the cell surface. A recent study (Tirosh et al., 1999) has examined the effect of peptide affinity on CTL response it elicits, either by a chemical modification, which renders peptide binding to the class I groove irreversible, or by optimizing the MHC anchor residues of the peptide. Working with the TAP-deficient RMA-S cells, it was shown that improving the affinity of a murine TAA-derived peptide could indeed result in significant enhancement of CTL induction and inhibition of tumor growth. However, at least in this particular system, there seems to exist an affinity ceiling, beyond which a corresponding augmentation in the magnitude of the immune response could not be achieved. An important observation in this study is of a significant decrease in the initial number of specific complexes, both of low and high affinity peptides, which occurred in the first two hours post-loading. This finding underlines an inherent limitation associated with the transient nature of MHC-binding by exogenous antigenic peptides, and reinforces the prospects of genetic modification of DCs.

A number of studies have indeed attempted to increase the actual frequency of the desired antigenic class I complexes on the cell surface, through genetic engineering of improved class I-peptide ligands. For example, one group (Mottez et al., 1995; Lone et al., 1998) has constructed a chimeric MHC class I molecule, in which the antigenic peptide was covalently linked to the amino terminal of the α chain. These proteins were expressed on the surface of transfected cells and were capable of eliciting a specific CTL response. However, using this approach, each antigenic peptide should be constructed with its own restricting α chain. To overcome this problem, another group (Uger and Barber, 1998) has attached the antigenic peptide to the amino terminal of the monomorphic β₂m. Primary T cells from mice, which had been immunized with the specific peptide, could indeed selectively lyse transfected cells, expressing these constructs. However, the cells used for expression in this study were deficient in MHC class I expression, due to a TAP transporter mutation. Yet, in spite of lack of competition from cytosol-derived peptides, level of peptide presentation was limited. Using a similar design, another study (Tafuro et al., 2001) has recently demonstrated reconstitution of MHC class I presentation in human cancer cells, but these, again, were class I-negative, due either to a TAP defect or to lack of β₂m expression. Although a non-mutated lymphoblastoid cell line was also included in this study and potentiated specific CTL lysis, there is no evidence as to the actual level of peptide presentation in these cells.

Citation or identification of any reference in any section of this application shall not be construed as an admission that such reference is available as prior art to the present invention.

### SUMMARY OF THE INVENTION

The present invention relates, in one aspect, to a polynucleotide comprising a sequence encoding a polypeptide that is capable of high level presentation of antigenic peptides on antigen-presenting cells, wherein the polypeptide comprises a β₂-microglobulin molecule that is linked through its carboxyl terminal to a polypeptide stretch that allows the anchorage of the β₂-microglobulin molecule to the cell membrane, and through its amino terminal to at least one antigeriic peptide comprising a MHC class I epitope, that is a tumorassociated antigen (TAA), wherein said polypeptide stretch at the β₂-microglobulin carboxyl terminal consists of a bridge peptide of about 10-15 amino acid residues within the membrane-proximal sequence of a class I heavy chain HLA molecule, which spans the whole distance to the cell membrane, said bridge peptide being linked to a sequence which can exert the required anchoring function being the full or partial transmembrane and/or cytoplasmic domain of a molecule selected from the group consisting of: (i) a human MHC class I molecule consisting of an HLA-A, HLA-B or HLA-C molecule; (ii) a costimulatory B7.1, B7.2 or CD40 molecule; and (iii) a signal transduction element capable of activating T cells or antigen-presenting cells. This chimeric polypeptide is referred to herein as "double-chimeric β₂-microglobulin" (dcβ₂m).

In one embodiment, an epitope, which is an antigenic determinant of one sole antigen, is linked to the amino terminal of the β₂-microglobulin. In another embodiment, there are two or more epitopes that may be antigenic determinants of the same or of two or more different antigens..

In another aspect, the present invention relates to a vector comprising a DNA molecule of the invention.

In a further aspect, the present invention relates to antigen-presenting cells (APCs), which express a dcβ₂m encoded by the DNA molecule of the invention as defined above. Any suitable professional APC can be used according to the invention such as dendritic cells, macrophages and B cells. In a preferred embodiment, the APC is a dendritic cell. Transfection or transduction of the cells is carried out by standard methods of recombinant DNA technology as well known to a person skilled in the art.

In one preferred embodiment, the APCs are capable of expressing a dcβ₂m polypeptide comprising at least one TAA peptide such as to present the TAA peptide(s) at a sufficiently high density to allow potent activation of peptide-specific cytotoxic T lymphocytes (CTL) capable of recognizing and binding to harmful tumor cells and causing their elimination or inactivation.

The present invention further provides a cancer vaccine comprising an agent selected from: (i) a DNA molecule encoding a dcβ₂m as defined herein wherein the at least one epitope linked to the amino terminal of β₂m is derived from at least one TAA; (ii) an expression vector comprising such DNA molecule (i); (iii) antigen presenting cells expressing a dcβ₂m as defined herein wherein the at least one epitope linked to the amino terminal of β₂m is derived from at least one TAA; (iv) antigen presenting cells (APCs) expressing a single-chimeric β₂-microglobulin (scβ₂m) molecule as defined herein consisting of a β₂microglobulin molecule linked through its carboxyl terminal to a polypeptide stretch that allows the anchorage of the β₂-microglobulin molecule to the cell membrane; and (v) APCs as defined in (iv) that have been pulsed with at least one TAA peptide.

The present invention still further provides pharmaceutical compositions for use in inducing a class I-restricted CTL response in a mammal comprising cells expressing a dcβ₂m of the invention.

### BRIEF DESCRIPTION OF THE FIGURES

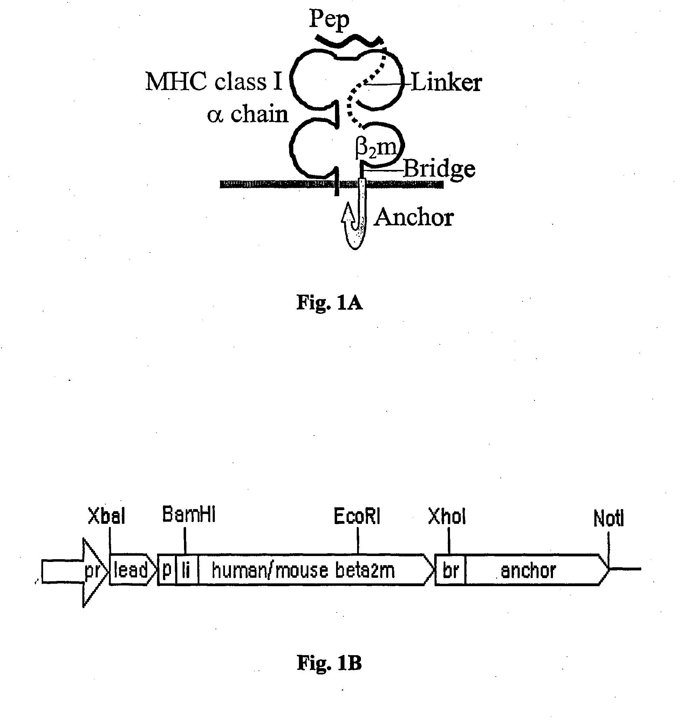

**Figs. 1A-1B** depict the construction and expression of dcβ₂m. **Fig. 1A** is a sketch of the dcβ₂m polypeptide associated on the cell surface with a MHC class I heavy (α) chain, while the antigenic peptide is in the binding groove. The transmembrane and cytoplasmic domains are derived from either the mouse CD3 ζ chain or the MHC class I heavy chain H-2K^{b}, and are covalently attached, via a short bridge, to the carboxyl teminus of human or mouse β₂m. The antigenic peptide is attached to the amino terminal of β₂m via a short linker with the sequence G₄S(G₃S)₂. **Fig. 1B** is a scheme of the genetic construct: pr, promoter; lead, leader peptide; p, antigenic peptide; li, linker peptide; br, bridge. Important restriction sites are indicated.

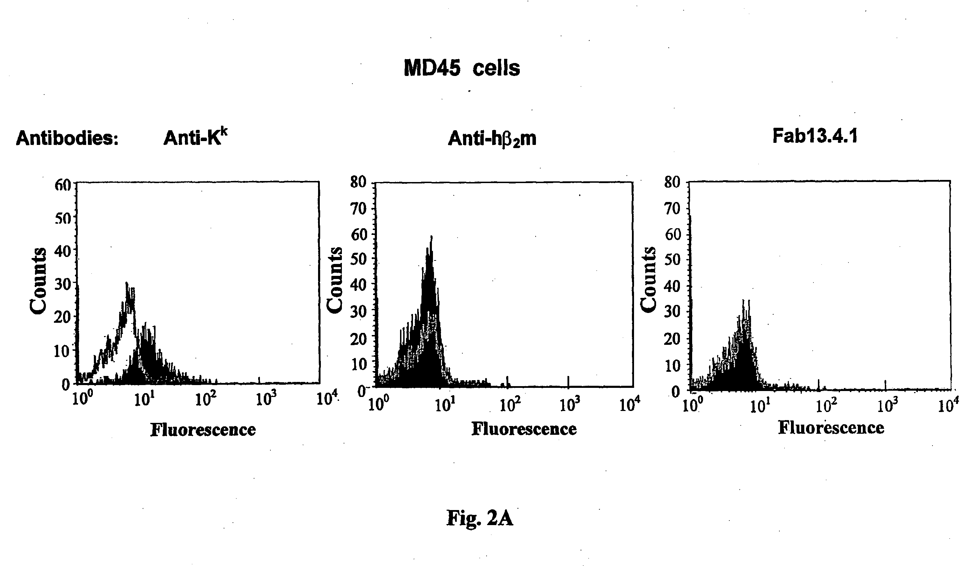

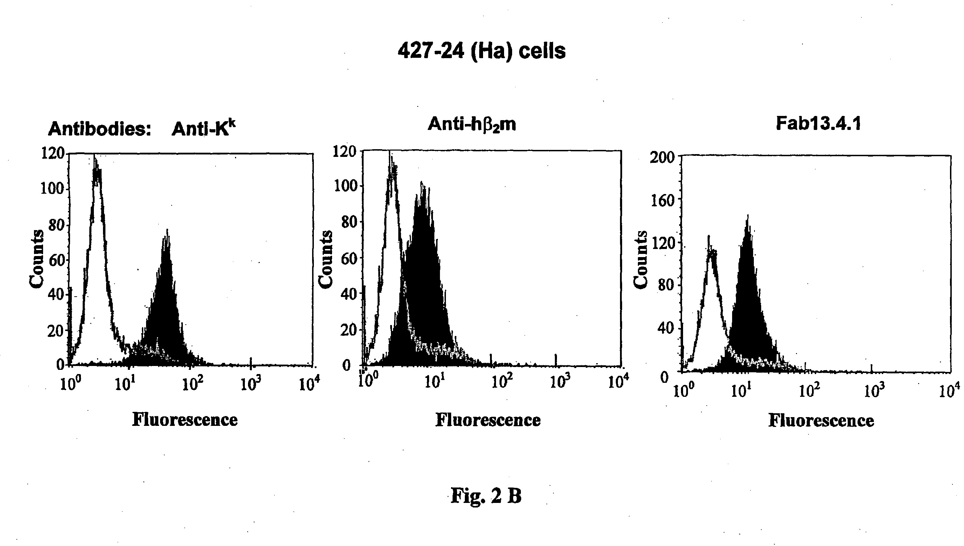

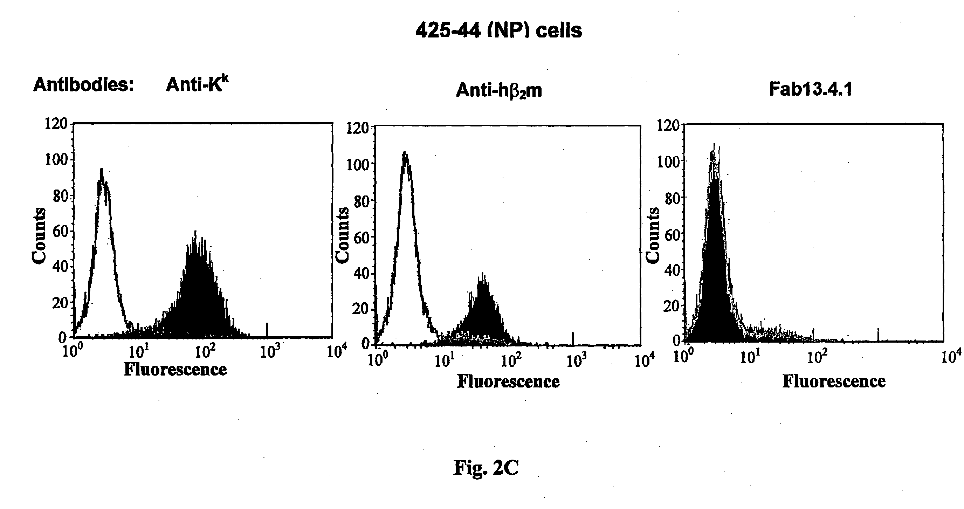

**Figs. 2A-2C** show flow cytometry analysis of MD45 parental cells **(****Fig. 2A****)** and transfectants 425-44 (NP) cells (expressing NP₅₀₋₅₇ dcβ₂m) **(****Fig. 2B****)** and 427-44 (Ha) cells (expressing the Ha₂₅₅₋₂₆₂ dcβ₂m) **(****Fig. 2C****).** Cells were analyzed with primary antibodies against H-2K^{k} (clone AF3-12.1), hβ₂m (clone BM-63) and K^{k}/Ha₂₅₅₋₂₆₂ complex (Fab13.4.1) and detected with secondary goat anti-mouse IgG (Fab-specific)-FITC conjugated polyclonal antibodies.

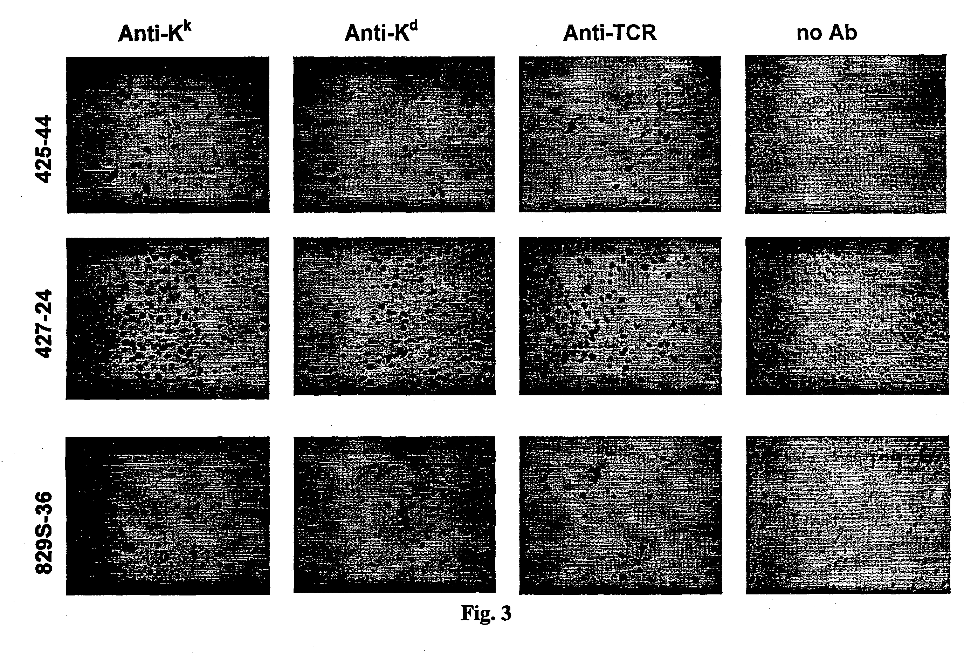

**Fig. 3** shows stimulation of the MD45 transfectants 425-44, 427-24 and 892S-36 (see Table 1 hereinafter) by different MHC-I allele-specific antibodies. Indicated cells at 5x10⁵/ml in 100 µl were incubated in wells of a microtiter plate pre-coated with the different antibodies at 5 µg/ml and then subjected to an in-cell X-Gal staining. Anti-K^{k} is AF3-12.1 and anti-K^{d} is SF1-1.1. Anti-TCR is the hamster anti-mouse CD3ε mAb 2C 11, which served as a positive control for activation.

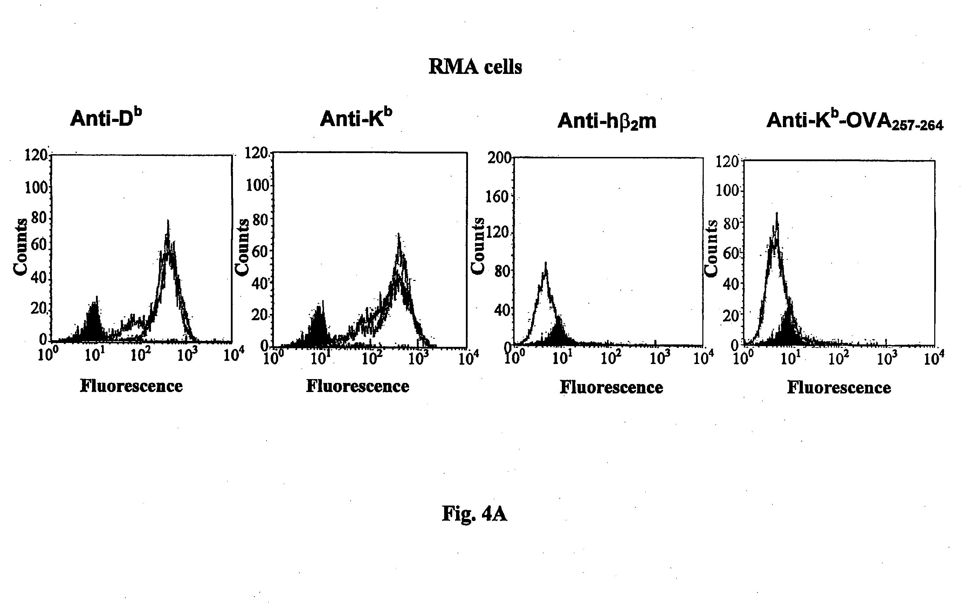

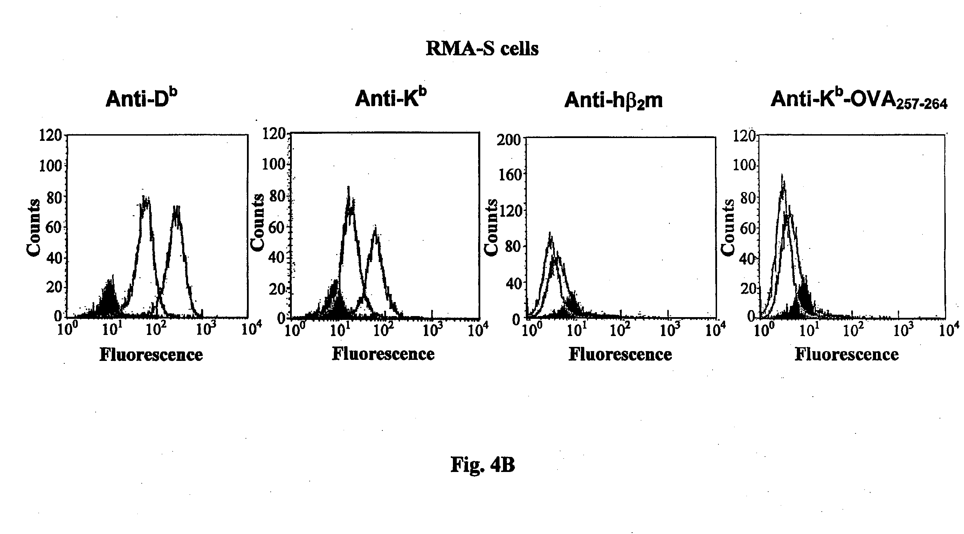

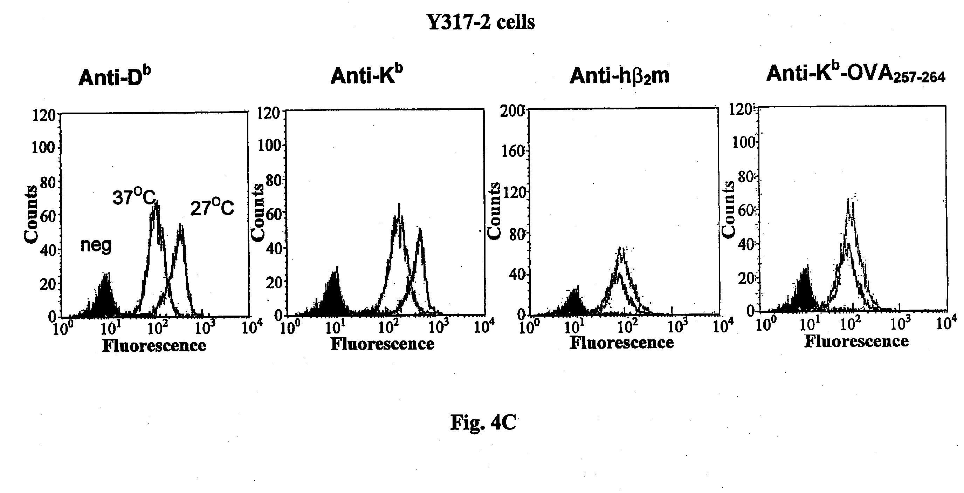

**Figs. 4A-4C** show FACS analysis of RMA **(****Fig. 4A****),** RMA-S **(****Fig. 4B****)** and transfectant Y317-2 (expressing OVA₂₅₇₋₂₆₄ linked to human membranal β₂m) cells **(****Fig. 4C****).** Antibodies were: anti-H-2Db (28-14-8); anti-H-2Kb (20-8-4); anti-hβ₂m (BM-63) and anti-k^{b}-OVA₂₅₇₋₂₆₄ (25-D1.16). Cells were grown for 24 hours in serum-free medium prior to staining at both 27°C and 37°C.

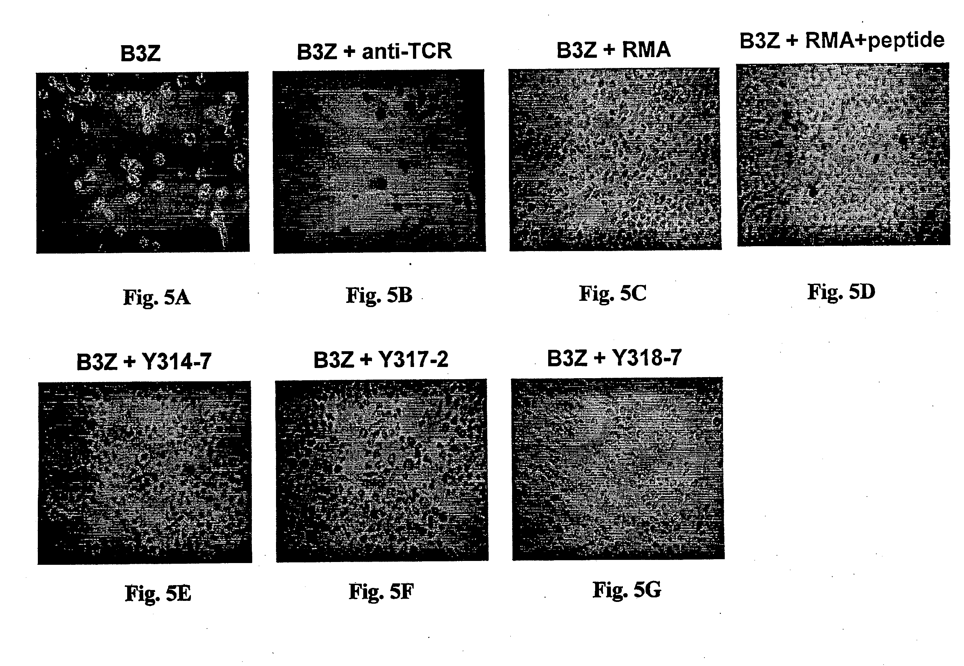

**Figs. 5A-5G** show that a K^{b}/OVA₂₅₇₋₂₆₄-specific T cell hybridoma is activated by cells expressing OVA₂₅₇₋₂₆₄ dcβ₂m. B3Z cells, an H-2K^{b}-restricted OVA₂₅₇₋₂₆₄-specific T cell hybridoma, were incubated with: 5A. no stimulation; 5B. Plastic-bound (5 µg/ml) anti-CD3ζ mAb (2C11); 5C. RMA cells; 5D. RMA cells loaded with synthetic OVA₂₅₇₋₂₆₄ at 2 µg/ml as a positive control; 5E. Y314-7 cells; 5F. Y317-2 cells; 5G. Y318-7 cells as a negative control. All cells were at 5x10⁵/ml. Cells were stained with X-Gal and visualized under a microscope.

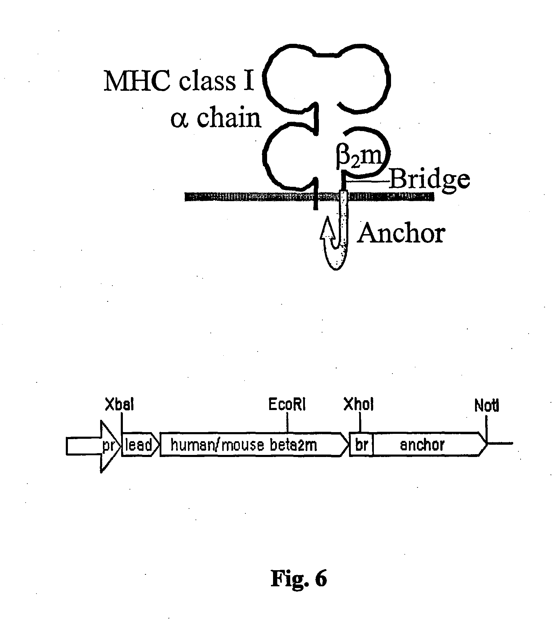

**Figs. 6A-6B** depict construction and expression of scβ₂m. **Fig. 6A** is a sketch of the scβ₂m polypeptide. The transmembrane and cytoplasmic domains are derived from either the mouse CD3 ζ chain or the MHC class I heavy chain H-2K^{b}, and are covalently attached, via a short bridge, to the carboxyl teminus of human or mouse β₂m. **Fig. 6B** is a scheme of the genetic construct: pr, promoter; lead, leader peptide; p, antigenic peptide; li, linker peptide; br, bridge. Important restriction sites are indicated.

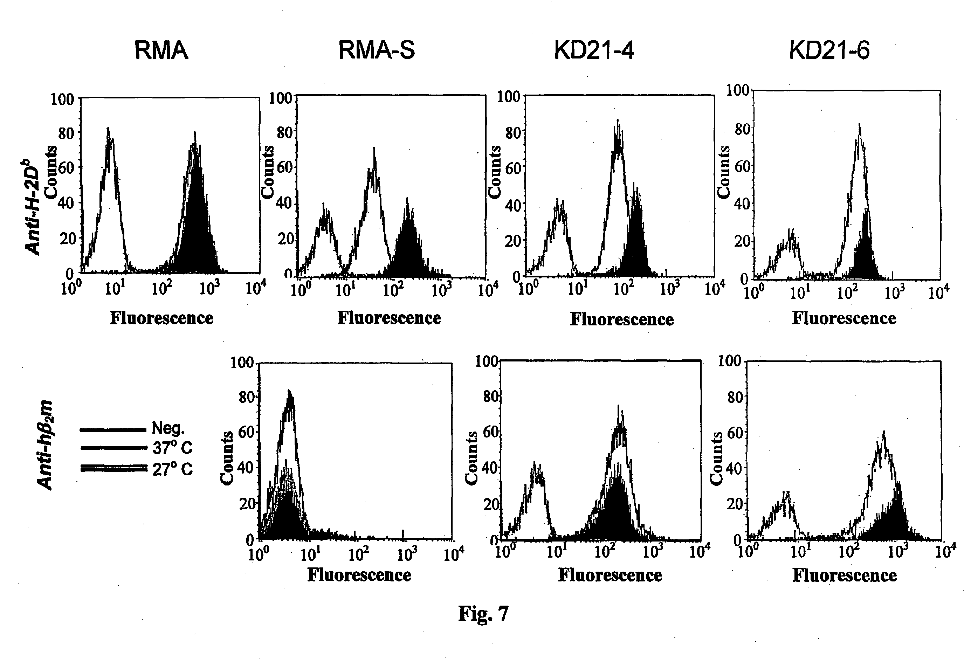

**Fig. 7** shows stabilization of MHC class I molecules by membranal β₂m. KD21-4 and KD21-6 RMA-S transfectants and parental RMA-S and RMA cells were grown in serum-free medium for 24 hours at 27°C and 37°C and then stained with anti-H-2Db (28-14-8) and anti-hβ₂m (BM-63) mAbs. FACS analysis was performed with FACSCalibur (BD Biosciences).

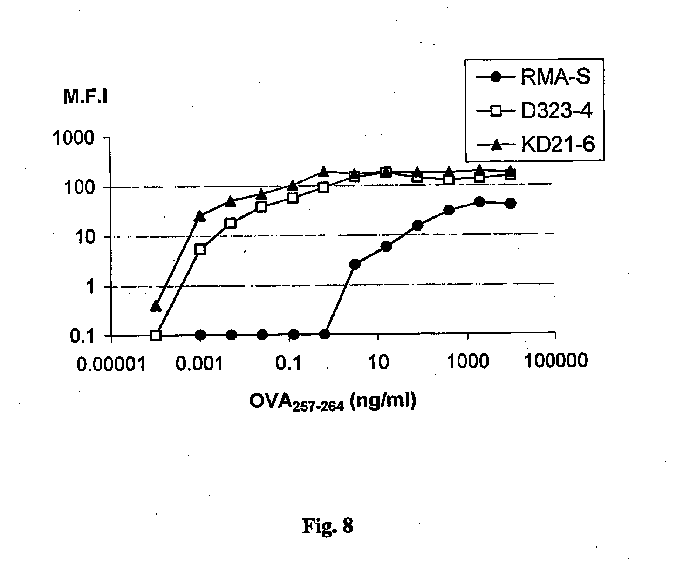

**Fig. 8** is a graph showing the ability of KD21-6 and D323-4 transfectants to bind exogenously added synthetic OVA₂₅₇₋₂₆₄ peptide through H-2K^{b}, in comparison with parental RMA-S cells. The cells were grown at 37°C for 24 hours in serum-free medium and were then incubated for 42 hours with serial dilutions of synthetic OVA₂₅₇₋₂₆₄. Cells were stained with mAb 25.D1-16 and FACS analysis was performed with FACSCalibur. Mean fluorescence intensity was calculated using CellQuest software.

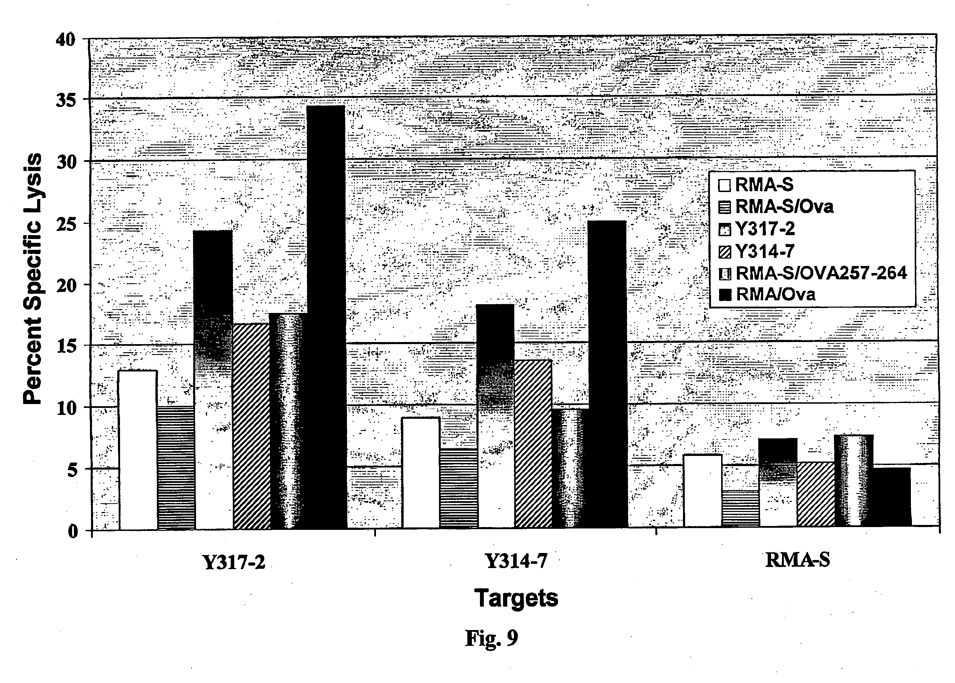

**Fig. 9** is a graph showing generation of antigen specific CTLs following cell immunization. RMA-S and RMA-S/OVA (negative controls), RMA-S loaded with OVA₂₅₇₋₂₆₄ and RMA/OVA (positive controls), and transfectants Y317-2 and Y314-7 were injected i.p. twice at 10-day interval. Ten days after the second immunization, CTLs were prepared and the indicated cells (Y317-2, Y314-7 and RMA-S) were used as target cells in a cell cytotoxicity assay at effector/target ratio of 50:1. Histogram shows percent specific lysis.

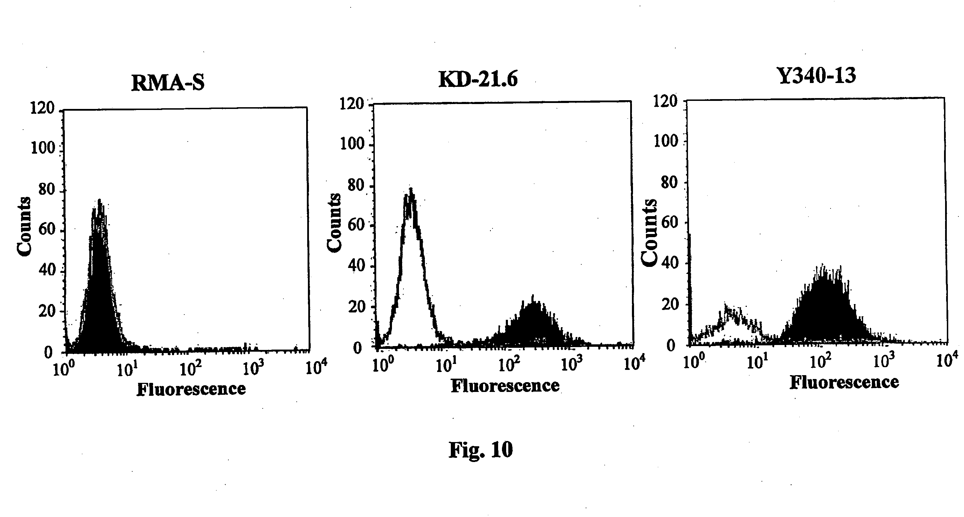

**Fig. 10** shows FACS analysis of RMA-S, KD21-6 and Y340-13 cells. Cells were analyzed with a primary antibody against hβ₂m (clone BM-63) and detected with secondary goat anti-mouse IgG (Fab-specific)-FITC conjugated polyclonal antibodies.

### DETAILED DESCRIPTION OF THE INVENTION

Duration of the functional MHC classI/peptide complex on the cell surface is governed by the affinity of the peptide for the MHC molecule. Dissociation of the peptide from its binding groove in the α heavy chain, results in practically irreversible disruption of the ternary complex formed between the α chain, β₂m and peptide. Both latter components are not anchored to the cell membrane and immediately detach from the cell, while the α chain is later internalized. Stabilization of a particular class I/peptide complex by enabling fast re-association is therefore likely to result in high level of presentation of the antigenic peptide.

In one aspect, the concept underlying the present invention is that connecting at least one epitope to one end (the amino terminal) of β₂m and anchoring this polypeptide to the cell membrane through its other end (the carboxyl terminal), will provide an exceedingly high level of the antigenic peptide directly to the ER in a TAP- and proteasome-independent manner and substantially increase complex stability, and consequently, the level of presentation of this peptide.

WO 01/91698 of the same applicants discloses the development by genetic engineering of a novel MHC class I configuration, in which the β₂m light chain is anchored to the cell membrane, while harboring an antigenic peptide related to an autoimmune disease fused to its amino terminal. Expression of this construct results in an exceptionally high level of the MHC-peptide complex on the surface of transfected cells, despite competition from normally presented peptides. Thus, an influenza virus hemagglutinin-derived peptide (Ha₂₅₅₋₂₆₂), restricted by the mouse class I allele K^{k}, was linked to the amino terminal of β₂m by genetic engineering, while the carboxyl terminal was anchored to the membrane of transfected, K^{k}-expressing cells. Analyses performed with an anti-K^{k} mAb and another mAb, which shows exquisite specificity to the K^{k}/Ha₂₅₅₋₂₆₂ complex, revealed high levels of the complex on the surface of transfected cells. It should be emphasized that efficient pairing of Ha₂₅₅₋₂₆₂ with K^{k} through this double-chimeric β₂m (dcβ₂ₘ) was achieved in-spite of strong competition from cytosolic-derived K^{k}-restricted peptides. Although data cannot be directly compared, it is to be noted that high level of surface class I-bound peptide by expression of non-membrane-attached β₂m/peptide alone, could not be directly demonstrated in previous studies (Uger and Barber, 1998; Tafuro et al., 2001). Membranal anchorage of dcβ₂m is therefore likely to result in substantial augmentation in the overall density of desired class I antigenic peptides on the cell surface, thus offering a novel and unique tool for CTL induction.

The main objectives of the present invention are to develop both a cell based-vaccine and a DNA vaccine, based on membranal β₂m carrying at least one antigenic peptide covalently bound to its amino terminal, wherein said antigenic peptide is a tumor-associated antigen (TAA).

As used herein, the terms "antigenic peptide" or "peptide or epitope derived from an antigen" mean both a peptide having a sequence comprised within the sequence of said antigen or an altered sequence, in which one or more amino acid residues have been replaced by different amino acid residues, which may bear higher affinity for the MHC class I molecule.

Thus, in one aspect, the present invention provides a polynucleotide comprising a sequence encoding a polypeptide that is capable of high level presentation of antigenic peptides on antigen-presenting cells, wherein the polypeptide comprises a β₂-microglobulin molecule that is linked through its carboxyl terminal to a polypeptide stretch that allows the anchorage of the β₂-microglobulin molecule to the cell membrane, and through its amino terminal to at least one antigenic peptide comprising a MHC class I epitope, that isa tumor-associated antigen (TAA), wherein saidpolypeptide stretch at the β₂-microglobulin carboxyl terminal consists of a bridge peptide of about 10-15 amino acid residues within the membrane-proximal sequence of a class I heavy chain HLA molecule, which spans the whole distance to the cell membrane, said bridge peptide being linked to a sequence which can exert the required anchoring function being the full or partial transmembrane and/or cytoplasmic domain of a molecule selected from the group consisting of: (i) a human MHC class I molecule consisting of an HLA-A, HLA-B or HLA-C molecule; (ii) a costimulatory B7.1, B7.2 or CD40 molecule; and (iii) a signal transduction element capable of activating T cells or antigen-presenting cells. In a most preferred embodiment, this bridge peptide is the peptide of SEQ ID NO: 1, of the sequence LRWEPSSQPTIPI.

In one embodiment, the anchoring residue (i) above is a sequence consisting of the transmembrane and cytoplasmic domains from the MHC class I heavy chain HLA-A2 molecule, of the SEQ ID NO: 2, of the sequence:

In another embodiment, the anchoring residue (iii) above is the intracellular region of a suitable signal transduction element capable of activating T cells such as, but not being limited to, a component of T-cell receptor CD3 such as the zeta (ζ) or eta (η) polypeptide, a B cell receptor polypeptide or an Fc receptor polypeptide. The cytoplasmic regions of the CD3 chains contain a motif designated the immunoreceptor tyrosine-based activation motif (ITAM), which has been shown to associate with cytoplasmic tyrosine kinases and to participate in signal transduction following TCR-mediated triggering. This motif is found in a number of other receptors including the Ig-α/Ig-β heterodimer of the B-cell receptor complex and Fc receptors for IgE and IgG, and three copies of it are found in the long cytoplasmic domains of the the ζ and η chains.

In a preferred embodiment, the anchoring residue of the chimeric molecule comprises the transmembranal and cytoplasmic regions of the human T-cell receptor CD3 ζ polypeptide, a signal transduction element capable of activating T cells.

In another embodiment, the signal transduction element capable of activating T cells comprises the transmembranal and cytoplasmic regions of a B-cell receptor polypeptide such as the Ig-α or Ig-β chain, the cytoplasmic tails in both being long enough to interact with intracellular signaling molecules. In a further embodiment, the signal transduction element comprises the transmembranal and cytoplasmic regions of Fc receptor polypeptides such as FcεRI, FcγRI or FcγRIII chains. FcεRI, a high-affinity receptor expressed on the surface of mast cells and basophils, contains four polypeptide chains: an α and a β chain and two identical disulfide-linked γ chains that extend a considerable distance into the cytoplasm and each has an ITAM motif. FcγRI, or CD64, is the high affinity receptor for IgG, expressed mainly on macrophages, neutrophils, eosinophils and dendritic cells. It comprises an α chain and two disulfide-linked γ chains. This structure is also typical to FcγRIII, or CD 16, which is the low affinity receptor for IgG, found on NK cells, eosinophils, macrophages, neutrophils and mast cells. CD3 ζ chain is found instead of the γ chain in a fraction of FcγRIII.

In still a further embodiment, the anchoring residue to which the bridge peptide is linked through its carboxyl terminal is a glycosylphosphatidylinositol (GPI)-anchor sequence, preferably the GPI-anchor peptide of SEQ ID NO:3, of the sequence FTLTGLLGTLVTMGLLT (from the protein DAF - complement decay-accelerating factor precursor or CD55 antigen; SWISSProt ID P08174, positions 365-381).

In one embodiment, the polynucleotide of the invention comprises a sequence encoding a polypeptide as defined in which the at least one antigenic peptide comprising a MHC class I epitope is linked to the β₂-microglobulin amino terminal directly. In another embodiment, the at least one antigenic peptide is linked to the β₂-microglobulin amino terminal through a peptide linker.

In one embodiment, the at least one antigenic peptide is at least one antigenic determinant of one sole antigen.

In another embodiment, the at least one antigenic peptide is at least one antigenic determinant of each one of at least two different antigens.

In one preferred embodiment of the invention, the at least one non-autoimmune disease related antigenic peptide comprising a MHC class I epitope linked to the β₂-microglobulin amino terminal is derived from a tumor-associated antigen (TAA) such as, but not limited to, alpha-fetoprotein, BA-46/lactadherin, BAGE (B antigen), BCR-ABL fusion protein, beta-catenin, CASP-8 (caspase-8), CDK4 (cyclin-dependent kinase 4), CEA (carcinoembryonic antigen), CRIPTO-1 (teratocarcinoma-derived growth factor), elongation factor 2, ETV6-AML1 fusion protein, G250/MN/CAIX, GAGE, gp100 gyp100 (glycoprotein 100)/Pme117, HER-2/neu (human epidermal receptor-2/neurological), intestinal carboxyl esterase, KIAA0205, MAGE (melanoma antigen), MART-1/Melan-A (melanoma antigen recognized by T cells/melanoma antigen A), MUC-1 (mucin 1), N-ras, p53, PAP (prostate acid phosphatase), PSA (prostate specific antigen), PSMA (prostate specific membrane antigen), telomerase, TRP-1/gp75 (tyrosinase related protein 1, or gp75), TRP-2, tyrosinase, and uroplakin Ia, Ib, II and III.

Examples of TAA peptides include, without being limited to, the following antigenic peptides:
- (i): the HLA-A2 restricted human alpha-fetoprotein peptide GVALQTMKQ (SEQ ID NO:4) associated with liver tumors;
- (ii): the HLA-Cw16 restricted human BAGE-1 peptide AARAVFLAL (SEQ ID NO:5);
- (iii): the HLA-A2 restricted human BCR-ABL fusion protein (b3a2) peptide SSKALQRPV (SEQ ID NO:6) associated with chronic myeloid leukemia;
- (iv): the HLA-A24 restricted human beta-catenin peptide SYLDSGIHF (SEQ ID NO:7) associated with melanoma;
- (v): the HLA-A2 restricted human CDK4 peptide ACDPHSGHFV (SEQ ID NO:8) associated with melanoma;
- (vi): the HLA-A2 restricted human CEA peptide YLSGANLNL (SEQ ID NO: 9) associated with gut carcinoma;
- (vii): the HLA-A68 restricted human elongation factor 2 peptide ETVSEQSNV (SEQ ID NO: 10) associated with lung squamous cell carcinoma;
- (viii): the HLA-A2 restricted human ETV6-AML1 fusion protein peptide RIAECILGM (SEQ ID NO:11) associated with acute lymphoblastic leukemia;
- (ix): the HLA-A2 restricted human G250 peptide HLSTAFARV (SEQ ID NO:12) associated with stomach, liver and pancreas tumors;
- (x): the HLA-Cw6 restricted human GAGE-1,2,8 peptide YRPRPRRY (SEQ ID NO:13);
- (xi): the gp 100 human peptides associated with melanoma HLA-A2 restricted KTWGQYWQV (SEQ ID NO:14), (A)MLGTHTMEV (SEQ ID NO:15), ITDQVPFSV (SEQ ID NO:16), YLEPGPVTA (SEQ ID NO:17), LLDGTATLRL (SEQ ID NO:18), VLYRYGSFSV (SEQ ID NO:19), SLADTNSLAV (SEQ ID NO:20), RLMKQDFSV (SEQ ID NO:21), RLPRIFCSC (SEQ ID NO:22), and the HLA-A3 restricted LIYRRRLMK (SEQ ID NO:23), ALLAVGATK (SEQ ID NO:24), IALNFPGSQK (SEQ ID NO:25) and ALNFPGSQK (SEQ ID NO:26);
- (xii): the HLA-A2 restricted human HER-2/neu ubiquitous peptide KIFGSLAFL (SEQ ID NO: 27);
- (xiii): the HLA-B7 restricted human intestinal carboxyl esterase peptide SPRWWPTCL (SEQ ID NO:28) associated with liver, intestine and kidney tumors;
- (xiv): the HLA-B44 restricted human KIAA0205 peptide AEPINIQTW (SEQ ID NO:29) associated with bladder tumor;
- (xv): the MAGE-1 peptides HLA-A1 restricted human EADPTGHSY (SEQ ID NO:30) and HLA-A3 restricted human SLFRAVITK (SEQ ID NO:31);
- (xvi): the MAGE-3 peptides HLA-A1 restricted human EVDPIGHLY (SEQ ID NO:32) and HLA-A2 restricted human FLWGPRALV (SEQ ID NO:33);
- (xvii): the HLA-A2 restricted human MART-1/Melan-A peptide (E)AAGIGILTV (SEQ ID NO:34) associated with melanoma;
- (xviii) the: HLA-A2 restricted human MUC-1 peptide STAPPVHNV (SEQ ID NO:35) associated with glandular epithelia carcinoma;
- (xix): the HLA-A1 restricted human N-ras peptide ILDTAGREEY (SEQ ID NO:36) associated with melanoma;
- (xx): the HLA-A2 restricted human p53 ubiquitous peptide LLGRNSFEV (SEQ ID NO:37);
- (xxi): the HLA-A2 restricted human PSA peptides FLTPKKLQCV (SEQ ID NO:38) and VISNDVCAQV (SEQ ID NO:39) associated with prostate carcinoma;
- (xxii): the HLA-A2 restricted human telomerase peptide ILAKFLHWL (SEQ ID NO: 40) associated with testis, thymus, bone marrow, and lymph nodes carcinomas;
- (xxiii) the: HLA-A31 restricted human TRP-1 peptide MSLQRQFLR (SEQ ID NO:41) associated with melanoma;
- (xxiv) the: HLA-A2 restricted human TRP-2 peptides LLGPGRPYR (SEQ ID NO:42), SVYDFFVWL (SEQ ID NO:43), and TLDSQVMSL (SEQ ID NO:44) associated with melanoma;
- (xxv): the HLA-A68 restricted human TRP2-INT2 peptide EVISCKLIKR (SEQ ID NO:45); and
- (xxvi): the HLA-A1 restricted human tyrosinase peptide KCDICTDEY (SEQ ID NO:46) associated with melanoma.

This list is presented only as examples of TAA peptides that can be used according to the invention. However, it is intended to encompass within the scope any TAA peptide known or to be discovered in the future as periodically published in Cancer Immunity, a Journal of the Academy of Cancer Immunology, at the website http://www.cancerimmunity.org/peptidedatabase/Tcellepitopes.htm.

In one embodiment of the invention, the polynucleotide encodes a polypeptide comprising at least one antigenic determinant of one sole TAA. In another preferred embodiment, the polynucleotide encodes a polypeptide comprising at least one antigenic determinant of each one of at least two different TAAs.

Thus, in some applications according to the invention, it may be desired to link more than one epitope to the amino terminal of the anchored β₂m. In this way, the product of a single DNA molecule can mediate the induction of CTL clones directed at different epitopes from the same TAA, or from two or more different TAAs, restricted by one or more HLA class I allelic products.

In one embodiment, the two or more epitopes may be derived from the same antigen. For example, at least 9 different HLA-A2 binding peptides and 4 different HLA-A3 binding peptides derived from the melanoma-associated antigen gp100 have been identified. A melanoma patient, who carries both HLA-A2 and HLA-A3, can, in principle, mount CTL responses to these 13 different gp 100-derived peptides.

Thus, in one preferred embodiment, the at least one antigenic peptide is at least one HLA-A2 binding peptide and at least one HLA-A3 binding peptide derived from the melanoma-associated antigen gp100, more preferably at least one gp100 HLA-A2 binding peptide selected from the group consisting of SEQ ID NO: 14, 15, 16, 17, 18, 19, 20, 21 and 22, and at least one gp100 HLA-A3 binding peptide selected from the group consisting of SEQ ID NO: 23, 24, 25 and 26.

In another embodiment, this strategy can be employed to elicit a CTL response to more than one antigenic molecule by using a single gene encoding epitopes of two different TAAs. For example, the same sequence can harbor peptides from gp100 and Melan-A/MART-1, both associated with melanoma, and harbor several HLA-A2-binding peptides, preferably at least one gp100 HLA-A2 binding peptide selected from the group consisting of SEQ ID NO: 14, 15, 16, 17, 18, 19, 20, 21 and 22, and at least one Melan-A/MART-1 HLA-A2 binding peptide selected from the group consisting of SEQ ID NO: 34. Similarly, peptides from different antigens, which bind different class I alleles can be incorporated on the same construct, e.g., HLA-A3-restricted gp100 and HLA-A2-restricted Melan-A/MART-1 peptide(s). Similarly, other combinations of different TAAs related to melanoma can be formed using one or more of the melanoma-associated TAAs described above, e.g. peptides derived from beta-catenin, CDK4, gp 100, Melan-A/MART-1, N-ras, TRP-1, TRP-2, and tyrosinase.

In another aspect, the present invention provides an expression vector comprising a polynucleotide of the invention as described hereinbefore.

Any suitable mammalian expression vector can be used such as, but not limited to, the pCI mammalian expression vectors (Promega, Madison, WI, USA), pCDNA3 expression vectors (Invitrogen, San Diego, CA) and pBJ1-Neo. The expression vector may also be a plasmid DNA in which the polynucleotide sequence is controlled by a virus, e.g. cytomegalovirus, promoter, or, most preferably, the expression vector is a recombinant viral vector such as, but not limited to, pox virus or adenovirus or adeno-associated viral vector.

In a further aspect, the present invention provides an antigen-presenting cell (APC) transfected with a polynucleotide comprising a sequence encoding a dcβ₂m of the invention.

The APC may be a macrophage, a B cell, a fibroblast and, more preferably, a dendritic cell..

In one embodiment, the at least one antigenic peptide in the antigen-presenting cell is at least one peptide derived from at least one TAA. Said cell is capable of presenting the at least one TAA peptide at a sufficiently high density to allow potent activation of peptide-specific cytotoxic T lymphocytes (CTL) capable of recognizing and binding to harmful tumor cells and causing their elimination or inactivation.

Any of the techniques which are available in the art may be used to introduce the recombinant nucleic acid encoding the polypeptide into the antigen presenting cell. These techniques are collectively referred to as transfection herein and include, but are not limited to, transfection with naked or encapsulated nucleic acids, cellular fusion, protoplast fusion, viral infection, cellular endocytosis of calcium-nucleic acid microprecipitates, fusion with liposomes containing nucleic acids, and electroporation. Choice of suitable vectors for expression is well within the skill of the art. Antigen expression may be determined by any of a variety of methods known in the art, such as immunocytochemistry, ELISA, Western blotting, radioimmunoassay, or protein fingerprinting.

In an additional aspect of the present invention, a DNA vaccine is provided comprising a polynucleotide of the invention or an expression vector of the invention, both as described hereinabove.

In one embodiment, there is provided a DNA vaccine for prevention or treatment of cancer comprising a polynucleotide that encodes a polypeptide comprising at least one antigenic determinant of at least one TAA.

The DNA vaccines may be constructed according to methods known in the art. Genes in plasmid expression vectors are expressed in vivo after intramuscular (i.m.) or subcutaneous (s.c.) injection and this expression stimulates an immune response against the plasmid-encoded proteins. The same or better effect is obtained replacing the plasmid by a viral vector.

In one embodiment, the DNA vaccine is a naked DNA vaccine. It may contain a plasmid DNA that contains the polynucleotide of the invention controlled by a cytomegalovirus (CMV) promoter. When the plasmid is introduced into mammalian cells, cell machinery transcribes and translates the gene. The expressed protein (immunogen) is then presented to the immune system where it can elicit an immune response. One method of introducing DNA into cells is by using a gene gun. This method of vaccination involves using pressurized helium gas to accelerate DNA-coated gold beads into the skin of the vaccinee.

DNA vaccines are capable of eliciting both strong humoral and cell-mediated immunity. Therefore DNA immunization represents a new approach for prevention (vaccination) and treatment (immune-based therapy) of infectious and neoplastic diseases.

In yet a further aspect of the invention, there is provided a cellular vaccine which comprises an antigen presenting cell of the invention as described hereinbefore. The antigen presenting cell is preferably a dendritic cell, but may also be a macrophage, a B cell and a fibroblast. The cells in the cellular vaccine may be autologous, allogeneic or xenogeneic cells.

The present invention provides cellular vaccines which comprise an antigen presenting cell that is capable of presenting at least one antigenic peptide comprising an epitope of at least one antigen and has the ability to induce potent CTL responses against the desired antigen(s). Vaccination, as used herein, refers to the step of administering the cellular vaccine to a mammal to induce such an immune responseto prevent or treat a tumor.

The presentation of the at least one antigenic peptide by the APCs in the cellular vaccine can be achieved by transfecting the APCs with the polynucleotide of the invention, or by transducing the APCs with a virus encoding the polynucleotide of the invention or by incubating said antigen presenting cells with a polynucleotide encoding said at least one antigenic peptide.

In one embodiment, the invention provides a cellular vaccine for prevention or treatment of cancer wherein the antigen presenting cell presents at least one peptide derived from at least one tumor associated antigen.

In an additional aspect, the present invention provides a cellular vaccine for the prevention and/or treatment of a cancer comprising antigen presenting cells which express a scβ₂m, i.e. a β₂-microglobulin linked through its carboxyl terminal to a polypeptide stretch that allows the anchorage of the β₂-microglobulin molecule to a cell membrane, wherein said polypeptide stretch consists of a bridge peptide which spans the whole distance to the cell membrane, said bridge peptide being linked to a sequence which can exert the required anchoring function, and wherein said cells have been pulsed with at least one antigenic peptide derived from at least one tumor associated antigen.

The cellular vaccine may be administered subcutaneously, intradermally, intratracheally, intranasally, or intravenously. The cells may be suspended in any pharmaceutically acceptable carrier, such as saline or phosphate-buffered saline.

In yet still a further aspect, the present invention provides pharmaceutical compositions. In one embodiment, the composition comprises as an active ingredient at least one polynucleotide or an expression vector of the invention, and a pharmaceutically acceptable carrier. The polynucleotide may comprise a sequence encoding a polypeptide comprising at least one antigenic peptide derived from at least one tumor associated antigen,. In another embodiment, the pharmaceutical composition comprises as an active ingredient at least one antigen presenting cell of the invention, and a pharmaceutically acceptable carrier.

Good cancer vaccines should induce a protective CTL response directed at MHC class I peptides derived from TAAs. The pivotal APC in CTL priming is the dendritic cell (DC), which has indeed been widely utilized in the design of cancer vaccines. In particular, DCs are attributed a critical role in DNA immunization, and direct presentation of peptides derived from expression of genetic material internalized by DCs is considered a major route for CTL induction. While magnitude of a CTL response correlates with density of specific MHC-peptide complexes on the APC surface, many TAA peptides have low affinity for the class I molecule and are presented at sub-optimal densities. Combined with the limiting expression level normally achieved following administration of non-replicating DNA, DNA immunization against TAAs usually falls short from achieving the anticipated effect.

The double-chimeric β₂m (dcβ₂m) polypeptide design of the present invention creates an entirely novel MHC class I entity, which may offer a great advantage over current strategies as a means to augment CTL induction. The membrane anchorage of the β₂m molecule can be achieved by covalently linking to its carboxyl terminal a peptide bridge, which spans the whole distance to the cell membrane, and is supplemented by an anchoring sequence such as the transmembrane and cytoplasmic domains derived from another cell surface protein. Following dissociation of β₂m-linked peptide from the α chain, this design is expected to prevent detachment of the β₂m/peptide from the cell membrane. Membrane anchorage should immensely increase the local concentration of dcβ₂m in the cell membrane, and allow rapid re-formation or de-novo formation of the specific MHC class I complex upon peptide dissociation. This will significantly prolong the actual half-life of the complex, and increase its membranal level.

Chimeric β₂m polypeptides having a sole antigenic peptide linked to their amino terminal, which are provided exogenously, have been shown to associate with α chains on the cell surface and to form full MHC class I complexes (Uger and Barber, 1998' Tafuro et al., 2001; Uger et al., 1999; White et al., 1999). According to the present invention, it is also assumed that re-association will take place on the cell membrane but obeying kinetics of lateral diffusion. Furthermore, but not less important, the high local peptide concentration, the membranal form of β₂m and the anticipated proteasome- and TAP-independence according to the invention, are all expected to render initial assembly of the specific, intact MHC class I complex in the ER highly favorable, compared with assembly involving processing and transportation of conventional, cytosolic peptides.

As used herein, the term "double-chimeric β₂-microglobulin" (dcβ₂m) refers to a molecule of β₂m having at least one epitope/antigenic peptide bound to the amino terminal and an anchor domain bound to the carboxyl terminal, wherein said anchor domain is composed of a polypeptide stretch consisting of a bridge peptide, which spans the whole distance to the cell membrane, and a peptide sequence that allows the anchorage of the β₂-microglobulin molecule to the cell membrane. The term "single-chimeric β₂-microglobulin" (scβ₂m), when used herein, refers to a molecule of β₂m having only the anchor domain, as defined above, but no antigenic peptide at the amino terminal.

The realization that vaccination with naked DNA results in long-lasting protein expression and stimulation of specific humoral and cellular immune responses, has made a large impact in the field of vaccine design (see Gurunathan et al., 2000 for review). Numerous studies, which have shown that DNA vaccines induce potent MHC class I-restricted CTL responses against TAAs, have suggested that this modality may be particularly useful for the treatment of cancer, and have prompted the development of a variety of DNA vaccine strategies (see review by Benton and Kennedy, 1998). First human trials of cancer DNA vaccines have been initiated, but it is too early to evaluate their efficacy. There is compelling evidence that a CTL response following DNA administration can be induced by directly transfected DCs (Porgador et al., 1998), although other mechanisms, such as direct transfection of somatic cells or cross presentation by DCs, are also considered.

According to the present invention, direct delivery of the dcβ₂ₘ polypeptide produced by DCs, which express the introduced gene, to surface MHC class I molecules for peptide presentation is expected to result in considerable enhancement in peptide level, and hence, in vaccine efficacy, compared with that achieved by conventional antigen processing and presentation.

The present invention thus provides a novel and broadly-applicable strategy for efficient induction of antigen-specific CTLs, which is based on the ability of dcβ₂m to markedly enhance presentation of antigenic peptides. The CTL response may be optimized by a regimen of two or more booster administrations. Cocktails of two or more CTL inducing peptides are employed to optimize epitope and/or MHC class I restricted coverage.

For the purposes of the present invention, the biochemical and immunological properties associated with this mode of presentation are first explored *in vitro* in transfected cell lines, and its *in vivo* function is then assessed in a mouse melanoma tumor model, applying transfected APC cell lines, naked DNA immunization and adoptive transfer of syngeneic APCs from transgenic mice.

Defining various parameters, which govern expression of dcβ₂m, and establishing its actual potential as a tumor vaccine in a mouse model are expected to pave the way for the design of a novel modality of human cancer vaccines. The most suitable effector cells for this purpose are autologous DCs, which can be relatively easily transduced to express foreign genes (Hadzantonis and O'Neill, 1999; Bubenik, 2001).

The inability to present low affinity peptides at densities required for potent activation of the entire repertoire of peptide-specific CTL clones is considered a major obstacle in many of the current protocols, which aim at producing DC-based cancer vaccines. According to the present invention, it is expected that the dcβ₂m-based constructs will increase the apparent affinity of the peptide to the MHC molecule and, thus, the dcβ₂m-mediated presentation on DCs should allow TAA-derived peptides with limiting affinity for the restricting MHC class I product to be presented by the DCs at sufficiently high density. This is one of the expected advantages of the present invention in comparison to previously proposed approaches for the development of cancer vaccines based on dendritic cells.

Some TAAs are expected to play an active part in the induction of central tolerance in the thymus, thus allowing only CTLs of low avidity to mature (Gilboa, 1999). These may include TAAs which are classified as differentiation antigens (for example MART-1/Melan A, gp100 and tyrosinase), and, probably to a lesser extent, normal gene products with highly restricted tissue distribution (such as MAGE, BAGE and GAGE). The strategy of the present invention can be efficient in activating such low avidity CTLs.

Tumors often evade the immune system by reduction in MHC class I peptide presentation to CTLs by downregulation of either components of the proteasome complex or TAP (for review see Benton and Kennedy, 1998). Enhancement of TAA peptide presentation by such tumors following gene delivery activates CTLs, which can respond also to non-modified tumor cells (Sherritt et al., 2001), provided the density of class I tumor-associated epitopes exceeds a functional threshold of these CTLs. Hence, dcβ₂M or scβ₂m are expected to induce CTLs not only in professional APCs as dendritic cells but, in certain cases, also when expressed in tumor cells.

The approach of the proposed invention offers broad applicability and requires only straightforward genetic engineering: since human β₂m is monomorphic, only one cloning expression cassette should be prepared, to which the segment encoding any antigenic peptide of interest can easily be inserted.

The invention will now be illustrated by the following non-limiting examples.

### EXAMPLES

### Materials and Methods

***(i) Cells.*** MD45 is an H-2D^{b}-allospecific mouse H-2^{k/d} CTL hybridoma of BALB/c origin (Faufmann et al., 1981). RMA-S is a mutant cell line derived from the C57BL/6 lymphoma RMA (H-2^{b}), which has defects in peptide presentation by class I MHC molecules due to loss of functional expression of the TAP component TAP-2. These cells can be loaded exogenously with high levels of MHC class I compatible peptides. RMA/OVA and RMA-S/OVA are clones of these two cells transfected with the full-length chicken ovalbumin gene. B3Z is an H-2K^{b}-restricted, OVA₂₅₇₋₂₆₄-specific CTL hybridoma, harboring the NFAT-LacZ reporter gene (Sanderson and Shastri, 1994), and is a gift from Dr. N. Shastri, University of California, Berkeley. Three clones of the mouse melanoma B16, a spontaneously-arising melanoma of C57BL/6 origin are used: F10.9 is a spontaneously metastasizing clone of the B16-F10 line, K1 is an H-2K^{b} transfectant of F10.9 (Porgador et al., 1989) and MO5 is a chicken ovalbumin-transfected variant of the B16 melanoma. C57BL/6-derived T cell Line A, reactive with TRP-2 peptide 181-188 (TRP-₂₁₈₁₋₁₈₈) (Bloom et al., 1997), is available from Dr. J. Yang, NCI, NIH, USA.
***(ii) Antibodies.*** Fab13.4.1 is a Fab fragment specific to Ha₂₅₅₋₂₆₂ in the context of K^{k}, and was a gift from Dr. J. Engberg, University of Copenhagen (Andersen et al., 1996). AF3-12.1 is an anti-K^{k} mAb (Pharmingen). BM-63 is an anti-human β₂m mAb (Sigma). 20.8.4 is an anti-H-2K^{b} mAb. 28-14-8 is an anti-H-2Db mAb. 25-D1.16 is specific to the complex H-2K^{b}/OVA₂₅₇₋₂₆₄ (Porgador et al., 1997). These latter antibodies are available from Dr. L. Eisenbach, Weizmann Institute of Science, Rehovot, Israel.
***(iii) DNA transfection.*** 5-10x10⁶ RMA-S cells in 0.8 ml were mixed in 4 mm sterile electroporation cuvette (ECU-104, EquiBio, Ashford, UK) with 10-20 µg DNA of the constructed plasmid and placed on ice. Transfection was performed by electroporation using Easyject Plus electroporation unit (EquiBio, Ashford, UK) at 350V, 750 µF. Cells were resuspended in fresh medium and cultured for 24-48 hours in 96-well plates prior to addition of the selecting drug (1 mg/ml G418). Resistant clones were first expanded in 24-well plates and analyzed for expression of the introduced gene by FACS.
***(iv) FACS analysis.*** Cells were stained with indicated antibodies according to standard procedures and were subjected to flow cytometry analysis. 10⁶ cells were washed with phosphate-buffered saline (PBS) containing 0.02% sodium azide and incubated for 30 minutes on ice with 100 µl of the anti-human β₂m mAb (Sigma) at 10 µg/ml or the same concentration of a control antibody (or no antibody). Cells were then washed and incubated on ice with 100 µl of 1:100 dilution of goat anti-mouse IgG (FAB specific)-FITC conjugated polyclonal antibody (Sigma) for 30 minutes. Cells were washed and resuspended in PBS and analyzed by a FACSCalibur (BD Biosciences, Mountain View, CA). Statistical analysis was performed with the FACSCalibur CellQuest software. Quantitative analysis of cell surface antigens was performed with QIFIKIT (DAKO, Carpinteria, CA) according to the manufacturer's instructions.
***(v) Cell stimulation assay.*** Cells at 5x10⁵ cells/ml were incubated overnight in 96-well plates in the presence of 5 µg/ml antibody (immobilized overnight and washed 3 times in PBS) or with target cells at 5x10⁵ cells/ml. Total volume: 0.1 ml.
***(vi) In-cell X-Gal staining.*** Cells in 96-well plates were washed twice with PBS and fixed with 0.25% glutaraldehyde for 15 min, washed 3 times in PBS, incubated for 4 hours with 100 µl of X-Gal solution {0.2% X-Gal, 2mM MgCl₂, 5mM K₄Fe(CN)₆ ·3H2O, 5mM K₃Fe(CN)₆ in PBS} and scored under the microscope for blue staining.
***(vii) Immunization of mice.*** Immunization was carried out with peptide loaded RMA-S cells, RMA-S, RMA-S/OVA and RMA/OVA and OVA₂₅₇₋₂₆₄-dcβ₂m-expressing RMA-S transfectants (Y314-7 and Y317-2): RMA-S cells were incubated at 2x10⁶ cells/ml for 2 hours with 200 µg/ml of OVA₂₅₇₋₂₆₄. Mice were immunized twice i.p. with 2x10⁶ irradiated (50 Gy) cells, at 10 day intervals.
***(viii) Cytotoxicity assay.*** Ten days after last immunization spleens were removed and single cell suspension were prepared. Splenocytes were restimulated with irradiated, mitomycin-C-treated tumor cells or target cells. Restimulated lymphocytes were maintained for another 4 days. Viable lymphocytes were separated on Lympholyte-M gradient (Cendarlane, Ontario, Canada) and resuspended at 5 x 10⁶/ml with lymphocyte medium. Lymphocytes were mixed at different ratios (1:100, 1:50, 1:25 and 1:12.5 target to effector) with ³⁵S-methionine-labeled target cells (tumor cells or peptide-presenting cells). CTL assays were carried out following standard procedures.

### Example 1. Expression of dcβ₂m designed for T cell re-programming

The general schemes of genetic constructs encoding dcβ₂m and of the polypeptide product associated with an MHC class I heavy chain are illustrated in Fig. 1. Table 1 summarizes all different single and double chimeric β₂m expression plasmids generated in this system as well in the tumor experimental system, which will be described below.

**Table 1. Double and single chimeric β₂m constructs and transfected clones expressing them**

| **Peptide** | **Allele** | β₂**2m** | **Anchor** | **Cell (H-2)** | **Clone** |
|---|---|---|---|---|---|
| IV Ha₂₅₅₋₂₆₂ | K^{k} | human | none | MD45(k/d) | 840-7 |
| IV Ha₂₅₅₋₂₆₂ | K^{k} | human | CD3 ζ | MD45 | 427-24 |
| IV NP₅₀₋₅₇ | Kk | human | CD3 ζ | MD45 | 425-44 |
| Insulin B₁₅₋₂₃ | Kd | mouse | CD3 ζ | MD45 | 829S-36 |
| OVA₂₅₇₋₂₆₄ | K^{b} | mouse | H-2K^{b} | RMA-S(b) | Y314-7 |
| OVA₂₅₇₋₂₆₄ | K^{b} | human | H-2K^{b} | RMA-S | Y317-2 |
| TRP-2₁₈₁₋₁₈₈ | K^{b} | mouse | H-2K^{b} | RMA-S | Y313-10 |
| TRP-2₁₈₁₋₁₈₈ | K^{b} | human | H-2K^{b} | RMA-S | Y318-7 |
| none | - | human | CD3 ζ | RMA-S | KD21-4,6 |
| none | - | human | H-2K^{b} | RMA-S | D323-4 |
| none | - | human | CD40 | B3Z(b) | Y340-13 |

In our previous patent application, WO 01/91698, it was aimed to redirect effector T cells against other, harmful T cells, through the CD3 ζ chain portion. In the experimental system described in WO 01/91698, two special mammalian expression cassettes were constructed, which allow the single-step insertion of a stretch coding for an antigenic peptide, so as to create dcβ₂m of either human or mouse origin. The bridging peptide, derived from the human MHC class I molecule HLA-A2, was the extracellular 13-amino acid stretch of SEQ ID NO:1, which is most proximal to the cell membrane, and the transmembrane and cytoplasmic domains were those of the mouse CD3 ζ chain. The sequence encoding the K^{k}-restricted influenza virus hemagglutinin peptide Ha₂₅₅₋₂₆₂ was cloned into the unique cloning sites in the human β₂m cassette. Plasmid DNA was transfected into the MD45 hybridoma, and one stable transfectant, designated 427-24 (Ha), was further analyzed. Another MD45 transfectant, designated 425-44 (NP), was generated, which similarly expresses the K^{k}-restricted influenza virus nucleoprotein peptide NP₅₀₋₅₇. FACS analysis was performed with the anti-hβ₂m and anti-H-2K^{k} antibodies and with the e/Ha₂₅₅₋₂₆₂ complex-specific Fab13.4.1. **Fig. 2** shows intensive staining of 427-24, but no detectable staining of the control cell 425-44 or of the parental MD45. Quantitative analysis of antigen level on the surface of both transfectants and parental MD45 cells is shown in **Table 2** and reveals occupation of 20% of surface H- K^{k} molecules of 427-24 cells by the Ha2₅₅₋₂₆₂ peptide.

**Table 2. Quantitative analysis of surface antigens of transfectants 425-44 and 427-24 and parental MD45 cells***

| | Antibody | | |
|---|---|---|---|
| Cell | Anti-H-2K^{k} | Anti-hβ2m | Fab13.4.1 |
| MD45 | 10,909 | 0 | 0 |
| 425-44 | 37,604 | 466,704 | 0 |
| 427-24 | 28,637 | 173,143 | 5,715 |

| | | | |
|---|---|---|---|
| * Cells were stained with the anti-H-2K^{k} mAb AF3-12.1, the anti-hβ₂m mAb BM-63 and Fab13.4.1, specific to the K^{k}/Ha₂₅₅₋₂₆₂ complex, and analyzed with QIFIKIT (Dako), using goat anti-mouse IgG (Fab-specific)-FITC conjugated polyclonal antibodies. Mean fluorescence intensities were derived with FACSCalibur software and standard curve was generated from the linear regression of five points at 3,600, 16,000, 53,000, 218,000 and 620,000 mouse IgG molecules per bead, using Excel. | | | |

It should be noted that the complex-specific antibody (Fab13.4.1) is a Fab, whereas the anti-H-2K^{k} is an intact IgG. Therefore, the actual occupation of H-2K^{k} molecules on the surface of 427-24 may in fact be higher. Also noteworthy is the 3-fold increase in the total amount of H-2K^{k} in both transfectants 425-44 and 427-24, compared with the parental MD45 cells.

It is conceivable that, on the cell surface, dcβ₂m polypeptides can associate with MHC class I allelic products other than the restricting one. In this scenario, the flexible peptide linker allows the covalently linked antigenic peptide to be situated away from the MHC binding groove, which is occupied by a conventional peptide. In order to test this structural prediction we designed a functional assay, based on the ability of our transfectants to respond to stimulation by Lac-Z production. If this indeed occurs, cells expressing an H-2K^{k} binding peptide will also be activated by an anti-H-2K^{d} mAb, and vice-versa. As shown in **Fig. 3****,** this is really the case. This finding implies to an elevated pool of membranal β₂m, which can become available by lateral diffusion for binding to their cognate MHC class I alleles following dissociation of their original peptide.

### Example 2. Construction and expression of dcβ₂m molecules harboring antigenic peptides of the B16 mouse melanoma model

The APCs for the animal studies are based on the commonly used RMA and RMA-S H-2^{b} cell lines. In the animal experiments, focus is on a mouse melanoma expressing a natural K^{b}-restricted, TAA-derived peptide, and, as a control for peptide specificity, a derivative of the same mouse melanoma is employed presenting another, highly immunogenic K^{b}-restricted peptide, following DNA transfection.

B16 is a spontaneous murine (m) melanoma originating in C57BL/6 mice. B16-F10.9 is a high metastatic line of B16, which shows a low cell surface expression of H-2K^{b}, and K1 is a low metastatic B16 variant, expressing high level of H-2K^{b} following DNA transfection (Porgador et al., 1989). TRP-2 was recently identified as a tumor rejection antigen for the B16 melanoma (Bloom et al., 1997). TRP-2₁₈₁₋₁₈₈, (VYDFFVWL - the peptide of SEQ ID NO: 43, in which the residue S at the amino terminal is absent) is a K^{b}-restricted peptide from TRP-2, and is a major peptide epitope in the induction of tumor-reactive CTLs, which mediate tumor rejection. MO5 is a chicken ovalbumin-transfected variant of the B16 melanoma. It presents the peptide OVA₂₅₇₋₂₆₄ (SIINFEKL - SEQ ID NO: 48), possessing H-2K^{b} anchor residues F at position 5 and L at position 8) in the context of K^{b}.

For further studies, including the B16 model, we replaced both the peptide bridge and the transmembrane and cytoplasmic domains of membranal β₂m with those of the H-2K^{b} molecule. A new XhoI/NotI fragment (see **Fig.1**), encoding this polypeptide stretch, was produced, bearing the DNA sequence of SEQ ID NO: 49:

From the 11^{th} codon (gcg) till the end this sequence encompasses the intact transmembrane and cytoplasmic portion of H-2K^{b} (positions 658-852 in GenBank accession J00400). The bridge is LRWEPSSSTVSNM (SEQ ID NO: 50), a fusion between the connecting peptide of HLA-A2 (at the carboxyl terminal) and H2-K^{b}. It is encoded by the sequence ctg aga tgg gag ccC TCG AGc tcc act gtc tcc aac atg, (SEQ ID NO: 51) with an XhoI site incorporated into the sequence.

The sequence of the sense primer comprises 2b protection and an XhoI site followed by the 3' part of the H-2K^{b} connecting peptide"
5' CCC TCG AGC TCC ACT GTC TCC AAC ATG GCG 3' (SEQ ID NO: 52)

The sequence of the reverse primer comprises 3b protection, a NotI site and it corresponds to GenBank accession J00400 positions 858-875:
5' CGC GCGG CCGC AAG TCC ACT CCA GGC AGC 3'(SEQ ID NO: 53)

The fragment was produced by RT-PCR performed on mRNA prepared from RMA (H-2^{b}) cells.

The sequences encoding both Trp-2₁₈₁₋₁₈₈ and OVA₂₅₇₋₂₆₄ were cloned as XbaI/BamHI fragments (see Fig. 1) with synthetic oligonucleotides, which were used for PCR amplification of the gene segments encoding mβ₂m leader peptide.

The sequence of the sense primer is:
5' GCG TCT AGA GCT TCA GTC GTC AGC ATG GCT CGC 3' (SEQ ID NO: 54)

It comprises 3b protection, an XbaI site and positions 38-61 in GenBank accession X01838, composed of 15 b 5' non- translated region of mβ₂m leader and the first 3 leader codons, including the ATG.

The sense sequence of the reverse primer for TRP-2₁₈₁₋₁₈₈ is:

It corresponds to the last 6 codons of the mβ₂m leader, the 8 codons for TRP-2₁₈₁₋₁₈₈ (GBA X66349 945-968), the first 5 codons of the linker peptide and 3b protection.

The final (reverse complementary sequence) is:

The sense sequence of the reverse primer for OVA₂₅₇₋₂₆₄ is:

It corresponds to the last 6 codons of the mβ₂m leader, the 8 codons for the OVA₂₅₇₋₂₆₄ (GenBank accession J00895, positions 7870-7893), the first 5 codons of the linker peptide and 3b protection.

The final (reverse complementary) sequence is:

As a BamHI/XhoI fragment encoding the carboxyl terminal of the linker peptide, the full mature hβ₂m and the amino terminal of the bridge we used the same fragment described in WO 01/91698. We created a similar fragment encoding mβ₂m, with the sequence of SEQ ID NO: 59:

From the 11^{th} codon (atc) till the 8^{th} codon before the end (atg) it encompasses positions 113-409 in GenBank accession X01838.

The sequence of the sense primer is:

It comprises 3b protection, a BamHI site, a segment encoding the carboxyl terminal of the linker peptide and the first 7 codons of the mature mβ₂m.

The sequence of the reverse primer is:

It comprises 4b protection, an XhoI site and it corresponds the last 7 codons of mβ₂m and to the amino terminal part of the bridge.

RT-PCR for amplification of mβ₂m sequences was performed on mRNA prepared from MD45 cells. Following verification of DNA sequences, each of the two XbaI/BamHI fragments was cloned into either pCI-Neo or pBJ1-Neo expression vectors, together with the BamHI/XhoI and the XhoI/NotI fragments described herein.

Stable transfectants with the resulting plasmids were generated and are listed in **Table 1.**

In order to evaluate expression of the new dcβ₂m constructs, we performed the FACS analysis shown in **Fig. 4****.** In this experiment, we compared expression of different MHC class I components on RMA, RMA-S and Y317-2 cells (transfected with OVA₂₅₇₋₂₆₄ fused to membranal hβ₂m), both at 37°C and 27°C. At this lower temperature MHC class I molecules on the TAP-deficient RMA-S cells are stabilized and their cell surface level increases. It is evident from this analysis that level of both H-2K^{b} and H-2D^{b} is considerably higher in Y317-2 cells than in their parental RMA-S cells. These results support our previous ones **(****Fig. 3****)** in showing that the chimeric polypeptide can associate on the cell surface with allelic products (in this case H-2D^{b}) other than the one binding the encoded antigenic peptide (K^{b}). Surface expression of the antigenic K^{b}/OVA₂₅₇₋₂₆₄ complex (as judged by staining with the 25D-1.16 mAb) conclusively indicates that presentation is TAP-independent. Comparison of mean fluorescence intensity (MFI) of expression at 37°C is presented in **Table 3** and reveals 57% H-2K^{b} occupancy in the transfectant.

**Table 3. Mean fluorescence intensities of clone Y317-2 stained with an allele-specific and complex-specific mAbs.**

| | **Antibody** | |
|---|---|---|
| **Cell** | Anti-H-2K^{b} | 25D-1.16 |
| Y317-2 | 121.7 | 69.7 |

We then went on to confirm that the linker peptide, which joins the carboxyl terminal of the antigenic peptide to the amino terminal of β₂m, does not interfere with T cell recognition. To this end we examined specific activation of B3Z, an H-2K⁶-restricted, OVA₂₅₇₋₂₆₄-specific CTL hybridoma, by RMA-S clones expressing dcβ₂m with OVA₂₅₇₋₂₆₄. The results, presented in **Fig. 5****,** show that the level of activation is indistinguishable from that achieved following incubation of parental RMA-S cells with synthetic OVA₂₅₇₋₂₆₄ and rule out major disruption of TCR-ligand interaction in this case.

### Example 3. Evaluating contribution of membrane anchorage of β₂m to MHC class I stability

In the experimental system described in WO 01/91698, a plasmid was assembled, designated 21-2, which encodes a membranal hβ₂m, linked to the transmembrane and cytoplasmic region of mouse CD3 ζ chain. Another plasmid, 323-3 was assembled, in which the CD3 ζ portion was replaced with those of H-2Kb. This was done as follows:
Scheme of genetic constructs encoding these single chimeric β₂m (scβ₂m) derivatives and of their expected polypeptide products associated with an MHC class I heavy chain are illustrated in **Fig. 6****.**

Plasmid 21-2 was introduced into RMA-S cells. Following FACS analysis of G418-resistant transfectants with the anti-hβ₂m antibody, two clones, designated KD21-4 and KD21-6, were chosen, the latter expressing higher level of membranal β₂m. These two clones were analyzed for the ability of the scβ₂m product to stabilize the MHC class I molecule H-2D^{b} at 37°C. Results of a typical experiment are presented in **Fig. 7****.** It is clear from these results that H-2D^{b} level is elevated at 37°C compared with the parental RMA-S cells, and that this elevation correlates with expression level of hβ₂m. In fact, for KD21-6, the level of surface H-2D^{b} is comparable to that of the wild-type RMA cells.

Plasmid 323-3 was similarly introduced to RMA-S cells and a stable transfectant, designated D323-4, which expresses high level of hβ₂m, was selected. In the next experiment we evaluated the ability of both KD21-6 and D323-4 transfectants to bind exogenously added synthetic OVA₂₅₇₋₂₆₄ peptide through H-2K^{b}, in comparison with parental RMA-S cells, exploiting the complex-specific 25D-1.16 mAb. This experiment was repeated 6 times, producing essentially identical results. Results of one of these experiments are shown in **Fig. 8****.** They demonstrate approximately 3 logs enhancement of the ability to bind exogenous peptide, while maximal level of binding increases only 3-4-fold compared with RMA-S cells. These findings imply that expression of the scβ₂m products results in a vast enhancement in the functional affinity of the antigenic peptide to the MHC class I molecule. It should be noted that the nature of the β₂m anchor (CD3 ζ in KD21-6 or H-2K^{b} in D323-4) has little influence on the magnitude of this striking phenomenon.

### Example 4. In-vivo assessment of dcβ₂m-based APCs

For in vivo evaluation of the capacity of dcβ₂m-based APCs to induce a specific CTL response, the RMA-S transfectants Y317-2 and Y314-7, expressing OVA₂₅₇₋₂₆₄ linked to hβ₂m or mβ₂m, respectively, were compared with cells exogenously loaded by peptides. In a preliminary experiment, C57BL/6 (B6) mice were immunized with the indicated cells. CTLs prepared from immunized mice were used in a cell cytotoxicity assay, in which transfectants were evaluated as target cells at various effector/target ratios. Results are depicted in **Fig. 9** and indicate that both Y317-2 and Y314-7 cells can serve as immunogens and as target cells for CTLs. These finding reinforce our previous conclusion that dcβ₂ₘ is an efficient vehicle for presentation of pre-selected antigenic peptides and that the linker peptide does not interfere with T cell recognition.

### Example 5. Assembly and preliminary evaluation of β₂m fused to CD40 trans-membrane and cytoplasmic region

DC licensing requires engagement of the CD40L on the CD4 T cell with CD40 on the DC and is a mandatory step in the elicitation of many CTL responses We reasoned that supplementing β₂m with the intracellular portion of CD40 might trigger CD40 signaling upon encounter of DCs expressing these new dcβ₂m constructs with specific CTLs, circumventing CD4 T cell help. In other words, the CD40 signaling moiety can serve as an adjuvant in membranal β₂m-based vaccines. To test this idea we assembled a new scβ₂m expression plasmid (encoding hβ₂m according to the general scheme illustrated in **Fig. 6**), in which the encoded anchor comprises CD40 transmembrane and cytoplasmic portion. This was done as follows:
The bridge is LRWEPSSSTVSNM (SEQ ID NO:50), a fusion between the connecting peptide of H-K^{b} with that of HLA-A2, as in **Example 2.** The gene segment encoding mouse CD40 transmembrane and cytoplasmic region encompasses positions 588-878 in GenBank accession M83312 and its DNA sequence (SEQ ID NO: 62) is:

The sequence of the sense primer (SEQ ID NO: 63) is:

It comprises 2b protection, an XhoI site followed by the 3' part of the segment encoding the bridge and the first 19b encoding the CD40 portion.

The sequence of the reverse primer (SEQ ID NO: 64) is:
5' CGC GCG GCC GCG GTC AGC AAG CAG CCA TC 3'

It corresponds to a stretch downstream the CD40 stop codon (positions 901-918 in GenBank Accession M83312) and contains NotI and 3b protection.

Messenger RNA was prepared from the murine B cell lymphoma A20, known to express CD40, and RT-PCR was performed with the two primers. The 369 bp product was cloned into pGEMT and DNA sequence was confirmed. The XhoI-NotI fragment was excised and inserted into the expression vector pBJ1-Neo cut with XbaI and NotI, together with the XbaI-XhoI fragment from plasmid 21-2, encoding hβ₂m with its leader peptide and the amino terminal of the bridge.

In order to assess function of the CD40 domains, we took advantage of the finding that CD40 can activate the nuclear factor of activated T cells (NFAT) (Choi et al., 1994). Plasmid DNA was introduced into B3Z cells (capable of high LacZ expression following stimulation through the NFAT-LacZ reporter gene) and resulting clones were screened for hβ₂m expression. FACS analysis, shown in **Fig. 10****,** reveals high expression of hβ₂m in one of the transfectants (Y340-13) but none in the parental B3Z cells.

### REFERENCES

Andersen, P. S., Stryhn, A., Hansen, B. E., Fugger, L., Engberg, J., and Buus, S. 1996. A recombinant antibody with the antigen-specific, major histocompatibility complex-restricted specificity of T cells. Proc Natl Acad Sci U S A 93:1820-4.
Armstrong, T. D., Pulaski, B. A., and Ostrand_Rosenberg, S. 1998. Tumor antigen presentation: changing the rules. Cancer Immunol. Immunother. 46:70-4.
Benton, P. A. and Kennedy, R. C. 1998. DNA vaccine strategies for the treatment of cancer. Curr Top Microbiol Immunol 226:1-20.
Berger, C. L., Longley, B. J., Imaeda, S., Christensen, I., Heald, P., and Edelson, R. L. 1998. Tumor-specific peptides in cutaneous T-cell lymphoma: association with class I major histocompatibility complex and possible derivation from the clonotypic T-cell receptor. Int J Cancer 76:304-11.
Bloom, M. B., Perry-Lalley, D., Robbins, P. F., Li, Y., el-Gamil, M., Rosenberg, S. A., and Yang, J. C. 1997. Identification of tyrosinase-related protein 2 as a tumor rejection antigen for the B16 melanoma. J Exp Med 185:453-9.
Bubenik, J. 2001. Genetically engineered dendritic cell-based cancer vaccines (review). Int J Oncol. 18:475-8.
Chen, W., Rains, N., Young, D., and Stubbs, R. S. 2000. Dendritic cell-based cancer immunotherapy: potential for treatment of colorectal cancer? J Gastroenterol Hepatol 15:698-705*.*
Choi, M. S., Brines, R. D., Holman, M. J., and Klaus, G. G. 1994. Induction of NFAT in normal B lymphocytes by anti-immunoglobulin or CD40 ligand in conjunction with IL-4. Immunity 1:179-87.
Cohen, I. R. and Weiner, H. L. 1988. T-cell vaccination. Immunol Today 9:332-5.
Dalgleish, A. G. 2001. Current problems in the development of specific immunotherapeutic approaches to cancer. J.Clin. Pathol. 54:675-6.
Feldman M. and Eisenbach L. 1991. MHC class I genes controlling the metastatic phenotype of tumor cells. Semin Cancer Biol. Oct;2(5):337-46:
Gervois, N., Guilloux, Y., Diez, E., and Jotereau, F. 1996. Suboptimal activation of melanoma infiltrating lymphocytes (TIL) due to low avidity of TCR/MHC-tumor peptide interactions. J. Exp.. Med. 183:2403-7.
Gilboa, E., Nair, S. K., and Lyerly, H. K. 1998. Immunotherapy of cancer with dendritic-cell-based vaccines. Cancer Immunol, Immunother 46:82-7.
Gilboa, E. 1999. The makings of a tumor rejection antigen. Immunity 11:263-70.
Gong, J., Chen, D., Kashiwaba, M., and Kufe, D. 1997. Induction of antitumor activity by immunization with fusions of dendritic and carcinoma cells. Nature Medicine 3:558-61.
Gong, J., Nikrui, N., Chen, D., Koido, S., Wu, Z., Tanaka, Y., Cannistra, S., Avigan, D., and Kufe, D. 2000. Fusions of human ovarian carcinoma cells with autologous or allogeneic dendritic cells induce antitumor immunity. J Immunol 165:1705-11.
Guermonprez, P., Valladeau, J., Zitvogel, L., Thery, C., and Amigorena, S. 2002. Antigen presentation and T cell stimulation by dendritic cells. Annu Rev Immunol 20:621-67.
Gurunathan, S., Klinman, D. M., and Seder, R. A. 2000. DNA vaccines: immunology, application, and optimization*. Annu Rev Immunol 18:927-74.
Hadzantonis, M. and O'Neill, H. 1999. Review: dendritic cell immunotherapy for melanoma. Cancer Biother Radiopharm. 14:11-22.
Heath, W. R. and Carbone, F. R. 2001. Cross-presentation, dendritic cells, tolerance and immunity. Annual Review of Immunology 19:47-64.
Horig, H., Young, A. C., Papadopoulos, N. J., DiLorenzo, T. P., and Nathenson, S. G. 1999. Binding of longer peptides to the H-2Kb heterodimer is restricted to peptides extended at their C terminus: refinement of the inherent MHC class I peptide binding criteria. J Immunol 163:4434-41.
Jager, E., Jager, D., and Knuth, A. 2002. Clinical cancer vaccine trials. Curr Opin Immunol 14:178-82*.*
Janeway, C. A. and Bottomly, K. 1994. Signals and signs for lymphocyte responses. Cell 76:275-85.
Kaufmann, Y., Berke, G., and Eshhar, Z. 1981. Cytotoxic T lymphocyte hybridomas that mediate specific tumor-cell lysis in vitro. Proc Natl Acad Sci USA 78:2502-6.
Kumar, V., Tabibiazar, R., Geysen, H. M., and Sercarz, E. 1995. Immunodominant framework region 3 peptide from TCR V beta 8.2 chain controls murine experimental autoimmune encephalomyelitis. J Immunol 154:1941-50.
Kumar, V. and Sercarz, E. 2001. An integrative model of regulation centered on recognition of TCR peptide/MHC complexes. Immunol Rev 182:113-21.
Levitsky, V., Zhang, Q. J., Levitskaya, J., and Masucci, M. G. 1996. The life span of major histocompatibility complex-peptide complexes influences the efficiency of presentation and immunogenicity of two class I-restricted cytotoxic T lymphocyte epitopes in the Epstein-Barr virus nuclear antigen 4. Jf Exp Med 183:915-26.
Lone, Y. C., Motta, I., Mottez, E., Guilloux, Y., Lim, A., Demay, F., Levraud, J. P., Kourilsky, P., and Abastado, J. P. 1998. In vitro induction of specific cytotoxic T lymphocytes using recombinant single-chain MHC class I/peptide complexes. J Immunother 21:283-94.
Minev, B. R., Chavez, F. L., and Mitchell, M. S. 1999. Cancer vaccines: novel approaches and new promise. Pharmacol and Therapeut 81:121-39.
Moingeon, P. 2001. Cancer vaccines. Vaccine 19:1305-26.
Mottez, E., Langlade_Demoyen, P., Gournier, H., Martinon, F., Maryanski, J., Kourilsky, P., and Abastado, J. P. 1995. Cells expressing a major histocompatibility complex class I molecule with a single covalently bound peptide are highly immunogenic. J Exp Med 181:493-502.
Nouri-Shirazi, M., Banchereau, J., Fay, J., and Palucka, K. 2000. Dendritic cell based tumor vaccines. Immunol Letters 74:5-10.
Offner, H., Jacobs, R., Bebo, B. F., Jr., and Vandenbark, A. A. 1999. Treatments targeting the T cell receptor (TCR): effects of TCR peptide- specific T cells on activation, migration, and encephalitogenicity of myelin basic protein-specific T cells. Springer Semin Immunopathol 21:77-90.
Ojcius, D. M., Langlade-Demoyen, P., Gachelin, G., and Kourilsky, P. 1994. Role for MHC class I molecules in selecting and protecting high affinity peptides in the presence of proteases. J Immunol 152:2798-810.
Porgador, A., Snyder, D., and Gilboa, E. 1996. Induction of antitumor immunity using bone marrow-generated dendritic cells. J Immunol 156:2918-26.
Porgador, A., Feldman, M., and Eisenbach, L. 1989. H-2Kb transfection of B16 melanoma cells results in reduced tumourigenicity and metastatic competence. J Immunogen 16:291-303.
Porgador, A., Yewdell, J. W., Deng, Y., Bennink, J. R., and Germain, R. N. 1997. Localization, quantitation, and in situ detection of specific peptide-MHC class I complexes using a monoclonal antibody. Immunity 6:715-26.
Porgador, A., Irvine, K. R., Iwasaki, A., Barber, B. H., Restifo, N. P., and Germain, R. N. 1998. Predominant role for directly transfected dendritic cells in antigen presentation to CD8+ T cells after gene gun immunization. J Exp Med 188:1075-82.
Reis e Sousa, C. 2001. Dendritic cells as sensors of infection. Immunity 14:495-8.
Rosenberg, S. A. 1999. A new era of cancer immunotherapy: converting theory to performance. CA Cancer J Clin. 49:70-3, 65.
Sanderson, S. and Shastri, N. 1994. LacZ inducible, antigen/MHC-specific T cell hybrids. Int Immunol 6:369-76.
Saric, T., Chang, S. C., Hattori, A., York, I. A., Markant, S., Rock, K. L., Tsujimoto, M., and Goldberg, A. L. 2002. An IFN-gamma-induced aminopeptidase in the ER, ERAP1, trims precursors to MHC class I-presented peptides. Nat Immunol 3:1169-76*.*
Serwold, T., Gonzalez, F., Kim, J., Jacob, R., and Shastri, N. 2002. ERAAP customizes peptides for MHC class I molecules in the endoplasmic reticulum. Nature 419:480-3.
Sherritt, M., Cooper, L., Moss, D. J., Kienzle, N., Altman, J., and Khanna, R. 2001. Immunization with tumor-associated epitopes fused to an endoplasmic reticulum translocation signal sequence affords protection against tumors with down-regulated expression of MHC and peptide transporters. Int Immunol 13:265-71.
Snyder, H. L., Yewdell, J. W., and Bennink, J. R. 1994. Trimming of antigenic peptides in an early secretory compartment. J Exp Med 180:2389-94.
Snyder, H. L., Bacik, I., Yewdell, J. W., Behrens, T. W., and Bennink, J. R. 1998. Promiscuous liberation of MHC-class I-binding peptides from the C termini of membrane and soluble proteins in the secretory pathway. Eur J Immunol 28:1339-46.
Sogn, J. A. 1998. Tumor immunology: the glass is half full. Immunity 9:757-63.
Sogn, J. A. 2000. The status of tumor immunology and cancer immunotherapy. Introduction. Immunol Investig 29:81-4.
Steinman, R. M. 1989. Dendritic cells: clinical aspects. Res Immunol 140:911-8; discussion 918-26.
Stryhn, A., Pedersen, L. O., Holm, A., and Buus, S. 2000. Longer peptide can be accommodated in the MHC class I binding site by a protrusion mechanism. Eur J Immunol 30:3089-99.
Tafuro, S., Meier, U. C., Dunbar, P. R., Jones, E. Y., Layton, G. T., Hunter, M. G., Bell, J. I., and McMichael, A. J. 2001. Reconstitution of antigen presentation in HLA class I-negative cancer cells with peptide-beta2m fusion molecules. Eur J Immunol 31:440-9.
Takahashi, H., Nakagawa, Y., Yokomuro, K., and Berzofsky, J. A. 1993. Induction of CD8+ cytotoxic T lymphocytes by immunization with syngeneic irradiated HIV-1 envelope derived peptide-pulsed dendritic cells. Int Immunol 5:849-57.
Tirosh, B., el_Shami, K., Vaisman, N., Carmon, L., Bar_Haim, E., Vadai, E., Feldman, M., Fridkin, M., and Eisenbach, L. 1999. Immunogenicity of H-2Kb-low affinity, high affinity, and covalently-bound peptides in anti-tumor vaccination. Immunol Letters 70:21-8.
Tsomides, T. J., Aldovini, A., Johnson, R. P., Walker, B. D., Young, R. A., and Eisen, H. N. 1994. Naturally processed viral peptides recognized by cytotoxic T lymphocytes on cells chronically infected by human immunodeficiency virus type 1. J Exp Med 180:1283-93.
Uger, R. A. and Barber, B. H. 1998. Creating CTL targets with epitope-linked beta 2-microglobulin constructs. J Immunol 160:1598-605.
Uger, R. A., Chan, S. M., and Barber, B. H. 1999. Covalent linkage to beta2-microglobulin enhances the MHC stability and antigenicity of suboptimal CTL epitopes. J Immunol 162:6024-8.
Vora, A. R., Rodgers, S., Parker, A. J., Start, R., Rees, R C., and Murray, A. K. 1997. An immunohistochemical study of altered immunomodulatory molecule expression in head and neck squamous cell carcinoma. Brit J Cancer 76:836-44.
Watson, G. A. and Lopez, D. M. 1995. Aberrant antigen presentation by macrophages from tumor-bearing mice is involved in the down-regulation of their T cell responses. J Immunol 155:3124-34.
Wen, Y. J. and Lim, S. H. 1997. T cells recognize the VH complementarity-determining region 3 of the idiotypic protein of B cell non-Hodgkin's lymphoma. Eur J Immunol 27:1043-7.
White, J., Crawford, F., Fremont, D., Marrack, P., and Kappler, J. 1999. Soluble class I MHC with beta2-microglobulin covalently linked peptides: specific binding to a T cell hybridoma. J Immunol 162:2671-6.
York, I. A., Chang, S. C., Saric, T., Keys, J. A., Favreau, J. M., Goldberg, A. L., and Rock, K. L. 2002. The ER aminopeptidase ERAP1 enhances or limits antigen presentation by trimming epitopes to 8-9 residues. Nat Immunol 3:1177-84.
Zhang, W., Cao, X., and Huang, X. 1997. [In vivo induction of antitumor immune response by tumor cells fused with GM-CSF gene-modified dendritic cells]. Zhonghua Yi Xue Za Zhi. (Chinese). 77:39-42.

## Claims

1. A polynucleotide comprising a sequence encoding a polypeptide that is capable of high level presentation of antigenic peptides on antigen-presenting cells, wherein the polypeptide comprises a β₂-microglobulin molecule that is linked through its carboxyl terminal to a polypeptide stretch that allows the anchorage of the β₂-microglobulin molecule to the cell membrane, and through its amino terminal to at least one antigenic peptide comprising a MHC class I epitope that is a tumor-associated antigen (TAA), wherein said polypeptide stretch at the β₂-microglobulin carboxyl terminal consists of a bridge peptide of about 10-15 amino acids within the membrane-proximal sequence of a class I heavy chain HLA molecule, which spans the whole distance to the cell membrane, said bridge peptide being linked to a sequence which can exert the required anchoring function, being the full or partial transmembrane and/or cytoplasmic domain of a molecule selected from the group consisting of:
(i) a human MHC class I molecule selected from an HLA-A, HLA-B or HLA-C molecule;
(ii) a costimulatory B7.1, B7.2 or CD40 molecule; and
(iii) a signal transduction element capable of activating T cells or antigen-presenting cells.

2. The polynucleotide of claim I, wherein said bridge peptide is the peptide having the sequence of SEQ ID NO: 1.

3. The polynucleotide of claim 1, wherein said bridge peptide is linked to the transmembrane and cytoplasmic domains from the MHC class I heavy chain HLA-A2 molecule having the sequence of SEQ ID NO: 2.

4. The polynucleotide of claim 1, wherein said transduction element capable of activating T cells is selected from:
(i) a component of T-cell receptor CD3 such as the zeta (ζ) or eta (η) polypeptide, optionally comprising the transmembranal and cytoplasmic regions of the human CD3 ζ polypeptide;
(ii) a B cell receptor polypeptide; and
(iii) an Fc receptor polypeptide.

5. The polynucleotide of claim 1 or 2, wherein said bridge peptide is linked through its carboxyl terminal to a GPI-anchor sequence such as the peptide having the sequence of SEQ ID NO: 3.

6. The polynucleotide of anyone of claims 1 to 5, wherein said at least one antigenic peptide comprising a MHC class I epitope is linked to the β₂-microglobulin amino terminal through a peptide linker, and said at least one antigenic peptide may be at least one antigenic determinant of one sole antigen or at least one antigenic determinant of each one of at least two different antigens.

7. The polynucleotide of claim 6, wherein said antigen is a tumor-associated antigen (TAA) such as alpha-fetoprotein, BA-46/lactadherin, BAGE, BCR-ABL fusion protein, beta-catenin, CASP-8, CDK4, CEA, CRIPTO-1, elongation factor 2, ETV6-AML1 fusion protein, G250, GAGE, gp100, HER-2/neu, intestinal carboxyl esterase, KIAA0205, MAGE, MART-1/Melan-A, MUC-1, N-ras, p53, PAP, PSA, PSMA, telomerase, TRP-1/gp75, TRP-2, tyrosinase, and uroplakin 1a, 1b, II and III.

8. The polynucleotide of claim 7, wherein said antigenic peptide is selected from the group consisting of:
(i) the alpha-fetoprotein peptide having the sequence of SEQ ID NO: 4;
(ii) the BAGE-1 peptide having the sequence of SEQ ID NO: 5;
(iii) the BCR-ABL fusion protein peptide having the sequence of SEQ ID NO: 6;
(iv) the beta-catenin peptide having the sequence of SEQ ID NO: 7;
(v) the CDK4 peptide having the sequence of SEQ ID NO: 8;
(vi) the CEA peptide having the sequence of SEQ ID NO: 9;
(vii) the elongation factor 2 peptide having the sequence of SEQ ID NO: 10;
(viii) the ETV6-AML1 fusion protein peptide having the sequence of SEQ ID NO:11;
(ix) the G250 peptide having the sequence of EQ ID NO: 12;
(x) the GAGE-1,2,8 peptide having the sequence of SEQ ID NO: 13;
(xi) the gp100 peptides having the sequences of SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, S SEQ ID NO: 25 and SEQ ID NO:26;
(xii) the HER-2/neu peptide having the sequence of SEQ ID NO: 27;
(xiii) the intestinal carboxyl esterase peptide having the sequence of SEQ ID NO: 28;
(xiv) the KIAA0205 peptide having the sequence of SEQ ID NO: 29;
(xv) the MAGE-1 peptides having the sequences of SEQ ID NO: 30 and SEQ ID NO: 31;
(xvi) the MAGE-3 peptides having the sequences of SEQ ID NO: 32 and SEQ ID NO: 33;
(xvii) the MART -1/Melan-A peptide having the sequence of SEQ ID NO: 34;
(xviii) the MUC-1 peptide having the sequence of SEQ ID NO: 35;
(xix) the N-ras peptide having the sequence of SEQ ID NO: 36;
(xx) the p53 peptide having the sequence of SEQ ID NO: 37;
(xxi) the PSA peptides having the sequences of SEQ ID NO: 38 and SEQ ID NO: 39;
(xxii) the telomerase peptide having the sequence of SEQ ID NO: 40;
(xxiii) the TRP-1 peptide having the sequence of SEQ ID NO: 41;
(xxiv) the TRP-2 peptides having the sequences of SEQ ID NO: 42, SEQ ID NO: 43, and SEQ ID NO: 44;
(xxv) the TRP2-INT2 peptide having the sequence of SEQ ID NO: 45; and
(xxvi) the tyrosinase peptide having the sequence of SEQ ID NO: 46.

9. The polynucleotide of claim 7 or 8, wherein said at least one antigenic peptide is at least one antigenic determinant of one sole tumor-associated antigen.

10. The polynucleotide of claim 9, wherein said at least one antigenic peptide is at least one HLA-A2 binding peptide and at least one HLA-A3 binding peptide derived from the melanoma-associated antigen gp100.

11. The polynucleotide of claim 10, wherein said at least one HLA-A2 binding peptide derived from gp100 is selected from the group consisting of SEQ ID NO: 14, 15, 16, 17, 18, 19, 20, 21 and 22; and said at least one gp100 HLA-A3 binding peptide is selected from the group consisting of SEQ ID NO: 23, 24, 25 and 26.

12. The polynucleotide of claim 7 or 8, wherein said, at least one antigenic peptide is at least one antigenic determinant of each one of at least two different tumor-associated antigens.

13. The polynucleotide of claim 12, wherein said at least one antigenic peptide is at least one HLA-A2 binding peptide derived from each one of the melanoma associated antigens gp100 and Melan-A/MART-1, or at least one HLA-A3-restricted gp100 and at least one HLA-A2-restricted Melan-A/MART-1 peptide.

14. An expression vector, preferably a recombinant viral vector, comprising a polynucleotide according to anyone of claims 1 to 13.

15. An antigen-presenting cell selected from the group consisting of a dendritic cell, a macrophage, a B cell or a fibroblast, transfected with a polynucleotide comprising a sequence encoding a polypeptide comprising a β₂-microglobulin molecule that is linked through its carboxyl terminal to a polypeptide stretch that allows the anchorage of the β₂-microglobulin molecule to the cell membrane, and through its amino terminal to at least one antigenic peptide comprising a MHC class I epitope that is a tumor-associated antigen (TAA), wherein said polypeptide stretch at the β₂-microglobulin carboxyl terminal consists of a bridge peptide of about 10-15 amino acids within the membrane-proximal sequence of a class I heavy chain HLA molecule, which spans the whole distance to the cell membrane, said bridge peptide being linked to a sequence which can exert the required anchoring function, optionally being the full or partial transmembrane and/or cytoplasmic domain of a molecule selected from the group consisting of:
(i) a human MHC class I molecule selected from an HLA-A, HLA-B or HLAC molecule;
(ii) a costimulatory B7.1, B7.2 or CD40 molecule; and
(iii) a signal transduction element capable of activating T cells or antigen-presenting cells.

16. A DNA vaccine comprising a polynucleotide of anyone of claims 1 to 13 or an expression vector of claim 14.

17. A cellular vaccine which comprises an antigen-presenting cell of claim 15.

18. A cellular vaccine comprising antigen-presenting cells which express a β₂-microglobulin molecule linked through its carboxyl terminal to a polypeptide stretch that allows the anchorage of the β₂-microglobulin molecule to the cell membrane, wherein said polypeptide stretch at the β₂-microglobulin carboxyl terminal consists of a bridge peptide which spans the whole distance to the cell membrane, said bridge peptide of about 10-15 amino acids within the membrane-proximal sequence of a class I heavy chain HLA molecule, being linked to a sequence which can exert the required anchoring function, optionally being the full or partial transmembrane and/or cytoplasmic domain of a molecule selected from the group consisting of:
(i) a human MHC class I molecule selected from an HLA-A, HLA-B or HLAC molecule;
(ii) a costimulatory B7.1, B7.2 or CD40 molecule; and
(iii) a signal transduction element capable of activating T cells or antigen-presenting cells, wherein said cells have been pulsed with at least one antigenic peptide derived from at least one tumor associated antigen.

19. Antigen-presenting cells which express a β₂-microglobulin molecule linked through its carboxyl terminal to a polypeptide stretch that allows the anchorage of the β₂-Microglobulin molecule to the cell membrane, wherein said polypeptide stretch at the β₂-microglobulin carboxyl terminal consists of a bridge peptide of about 10-15 amino acids within the membrane-proximal sequence of a class I heavy chain HLA molecule, which spans the whole distance to the cell membrane, said bridge peptide being linked to a sequence which can exert the required anchoring function, optionally being the full or partial transmembrane and/or cytoplasmic domain of a molecule selected from the group consisting of:
(i) a human MHC class I molecule selected from an HLA-A, HLA-B or HLAC molecule;
(ii) a costimulatory B7.1, B7.2 or CD40 molecule; and
(iii) a signal transduction element capable of activating T cells or antigen-presenting cells, wherein said cells have been pulsed with at least one antigenic peptide derived from at least one tumor associated antigen for use in the prevention or treatment of cancer.

20. The cellular vaccine of claim 17 or 18, wherein the antigen-presenting cell is selected from the group consisting of a dendritic cell, a macrophage, a B cell and a fibroblast, wherein said antigen-presenting cell presents at least one antigenic peptide derived from at least one tumor associated antigen.

21. The antigen-presenting cells of claim 19, wherein the antigen-presenting cells are selected from the group consisting of dendritic cells, macrophages, B cells and fibroblasts, wherein said antigen-presenting cells present at least one antigenic peptide derived from at least one tumor associated antigen.

22. A pharmaceutical composition comprising as an active ingredient at least one polynucleotide of any one of claims 1 to 13 or an expression vector of claim 14, or an antigen-presenting cell of claim 15, and a pharmaceutically acceptable carrier.

23. The expression vector of claim 14, or the antigen-presenting cell of claim 15, for use in the prevention or treatment of cancer.

## Patentansprüche

1. Polynucleotid, umfassend eine Sequenz, die ein Polypeptid codiert, das fähig ist, auf antigenpräsentierenden Zellen einen hohen Spiegel an antigenen Peptiden zu präsentieren, wobei das Polypeptid ein β₂-Mikroglobulinmolekül umfasst, das über seinen Carboxy-Terminus an einen Polypeptidabschnitt gebunden ist, der die Verankerung des β₂-Mikroglobulinmoleküls an der Zellmembran ermöglicht, und über seinen Amino-Terminus an mindestens ein antigenes Peptid gebunden ist, das ein MHC-Klasse-l-Epitop umfasst, das ein Tumor-assoziiertes Antigen (TAA) ist, wobei der Polypeptidabschnitt am Carboxy-Terminus des β₂-Mikroglobulins aus einem Brückenpeptid von etwa 10-15 Aminosäuren innerhalb der membranproximalen Sequenz eines HLA-Klasse-I-Moleküls der schweren Kette besteht, das den vollständigen Abstand bis zur Zellmembran überspannt, wobei das Brückenpeptid an eine Sequenz gebunden ist, die die erforderliche Ankerfunktion ausüben kann und die die vollständige oder partielle Transmembran-Domäne und/oder cytoplasmatische Domäne eines Moleküls ist, das ausgewählt ist aus der Gruppe bestehend aus:
(i) einem menschlichen MHC-Klasse-I-Molekül ausgewählt aus einem HLA-A-, HLA-B- oder HLA-C-Molekül;
(ii) einem ko-stimulierenden B7.1-, B7.2- oder CD40-Molekül; und
(iii) einem Signaltransduktionselement, das zur Aktivierung von T-Zellen oder antigenpräsentierenden Zellen fähig ist.

2. Polynucleotid nach Anspruch 1, wobei das Brückenpeptid das Peptid ist, das die Sequenz der SEQ ID NO:1 aufweist.

3. Polynucleotid nach Anspruch 1, wobei das Brückenpeptid an die Transmembran-Domäne und cytoplasmatische Domäne des MHC-Klasse-I-HLA-A2-Moleküls der schweren Kette mit der Sequenz der SEQ ID NO:2 gebunden ist.

4. Polynucleotid nach Anspruch 1, wobei das Transduktionselement, das zur Aktivierung von T-Zellen fähig ist, ausgewählt ist aus
(i) einem Bestandteil eines T-Zellrezeptors CD3 wie das Zeta (ζ)- oder Eta (η)-Polypeptid, gegebenenfalls die Transmembran-und cytoplasmatischen Regionen des menschlichen CD3-ζ-Polypeptids umfassend;
(ii) einem B-Zellrezeptor-Polypeptid; und
(iii) einem Fc-Rezeptor-Polypeptid.

5. Polynucleotid nach Anspruch 1 oder 2, wobei das Brückenpeptid über seinen Carboxy-Terminus an eine GPI-Ankersequenz, wie das Peptid mit der Sequenz der SEQ ID NO:3, gebunden ist.

6. Polynucleotid nach einem der Ansprüche 1 bis 5, wobei das mindestens eine antigene Peptid, das ein MHC-Klasse-I-Epitop umfasst, über einen Peptidlinker an den Amino-Terminus des β₂-Mikroglobulins gebunden ist, und das mindestens eine antigene Peptid mindestens eine antigene Determinante eines einzigen Antigens oder mindestens eine antigene Determinante eines jeden von mindestens zwei verschiedenen Antigenen sein kann.

7. Polynucleotid nach Anspruch 6, wobei das Antigen ein Tumor-assoziiertes Antigen (TAA) ist, wie Alpha-Fetoprotein, BA-46/Lactadherin, BAGE, BCR-ABL-Fusionsprotein, Beta-Catenin, CASP-8, CDK4, CEA, CRIPTO-1, Elongationsfaktor 2, ETV6-AML1-Fusionsprotein, G250, GAGE, gp100, HER-2/neu, intestinale Carboxylesterase, KIAA0205, MAGE, MART-1/Melan-A, MUC-1, N-ras, p53, PAP, PSA, PSMA, Telomerase, TRP-1/gp75, TRP-2, Tyrosinase und Uroplakin 1a, 1b, II und III.

8. Polynucleotid nach Anspruch 7, wobei das antigene Peptid ausgewählt ist aus der Gruppe bestehend aus:
(i) dem Alpha-Fetoprotein-Peptid mit der Sequenz der SEQ ID NO:4;
(ii) dem BAGE-1-Peptid mit der Sequenz der SEQ ID NO:5;
(iii) dem BCR-ABL-Fusionsprotein-Peptid mit der Sequenz der SEQ ID NO:6;
(iv) dem Beta-Catenin-Petid mit der Sequenz der SEQ ID NO:7;
(v) dem CDK4-Peptid mit der Sequenz der SEQ ID NO:8;
(vi) dem CEA-Peptid mit der Sequenz der SEQ ID NO:9;
(vii) dem Elongationsfaktor-2-Peptid mit der Sequenz der SEQ ID NO:10;
(viii) dem ETV6-AML1-Fusionsprotein-Peptid mit der Sequenz der SEQ ID NO:11;
(ix) dem G250-Peptid mit der Sequenz der SEQ ID NO:12;
(x) dem GAGE-1,2,8-Peptid mit der Sequenz der SEQ ID NO:13;
(xi) den gp100-Peptiden mit den Sequenzen der SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25 und SEQ ID NO:26;
(xii) dem HER-2/neu-Peptid mit der Sequenz der SEQ ID NO:27;
(xiii) dem intestinalen Carboxylesterase-Peptid mit der Sequenz der SEQ ID NO:28;
(xiv) dem KIAA0205-Peptid mit der Sequenz der SEQ ID NO:29;
(xv) den MAGE-1-Peptiden mit den Sequenzen der SEQ ID NO:30 und SEQ ID NO:31;
(xvi) den MAGE-3-Peptiden mit den Sequenzen der SEQ ID NO:32 und SEQ ID NO:33;
(xvii) dem MART-1/Melan-A-Peptid mit der Sequenz der SEQ ID NO:34;
(xviii) dem MUC-1-Peptid mit der Sequenz der SEQ ID NO:35;
(xix) dem N-ras-Peptid mit der Sequenz der SEQ ID NO:36;
(xx) dem p53-Peptid mit der Sequenz der SEQ ID NO:37;
(xxi) den PSA-Peptiden mit den Sequenzen der SEQ ID NO:38 und SEQ ID NO:39;
(xxii) dem Telomerase-Peptid mit der Sequenz der SEQ ID NO:40;
(xxiii) dem TRP-1-Peptid mit der Sequenz der SEQ ID NO:41;
(xxiv) den TRP-2-Peptiden mit den Sequenzen der SEQ ID NO:42, SEQ ID NO:43 und SEQ ID NO:44;
(xxv) dem TRP2-INT2-Peptid mit der Sequenz der SEQ ID NO:45; und
(xxvi) dem Tyrosinase-Peptid mit der Sequenz der SEQ ID NO:46.

9. Polynucleotid nach Anspruch 7 oder 8, wobei mindestens ein antigenes Peptid mindestens eine antigene Determinante eines einzigen Tumor-assoziierten Antigens ist.

10. Polynucleotid nach Anspruch 9, wobei das mindestens eine antigene Peptid mindestens ein HLA-A2-bindendes Peptid ist und mindestens ein HLA-A3-bindendes Peptid ist, das vom Melanom-assoziierten Antigen gp100 stammt.

11. Polynucleotid nach Anspruch 10, wobei das mindestens eine von gp100 stammende HLA-A2-bindende Peptid ausgewählt ist aus der Gruppe bestehend aus SEQ ID NO:14, 15, 16, 17, 18, 19, 20, 21 und 22; und das mindestens eine gp100-HLA-A3-bindende Peptid ausgewählt ist aus der Gruppe bestehend aus SEQ ID NO:23, 24, 25 und 26.

12. Polynucleotid nach Anspruch 7 oder 8, wobei das mindestens eine antigene Peptid mindestens eine antigene Determinante eines jeden von mindestens zwei verschiedenen Tumor-assoziierten Antigenen ist.

13. Polynucleotid nach Anspruch 12, wobei das mindestens eine antigene Peptid mindestens ein HLA-A2-bindendes Peptid, das von jedem der Melanom-assoziierten Antigene gp100 und Melan-A/MART-1 stammt, oder mindestens ein HLA-A3-restringiertes gp100-Peptid oder mindestens ein HLA-A2-restringiertes Melan-A/MART-1-Peptid ist.

14. Expressionsvektor, bevorzugt ein rekombinanter viraler Vektor, der ein Poylnucleotid nach einem der Ansprüche 1 bis 13 umfasst.

15. Antigenpräsentierende Zelle, ausgewählt aus der Gruppe bestehend aus einer dendritischen Zelle, einem Makrophagen, einer B-Zelle oder einem Fibroblasten, transfiziert mit einem Polynucleotid, das eine Sequenz umfasst, die ein Polypeptid codiert, das ein β₂-Mikroglobulinmolekül umfasst, das über seinen Carboxy-Terminus an einen Polypeptidabschnitt gebunden ist, der die Verankerung des β₂-Mikroglobulinmoleküls an der Zellmembran ermöglicht, und über seinen Amino-Terminus an mindestens ein antigenes Peptid gebunden ist, das ein MHC-Klasse-I-Epitop umfasst, das ein Tumor-assoziiertes Antigen (TAA) ist, wobei der Polypeptidabschnitt am Carboxy-Terminus des β₂-Mikroglobulins aus einem Brückenpeptid von etwa 10-15 Aminosäuren innerhalb der membranproximalen Sequenz eines HLA-Klasse-I-Moleküls der schweren Kette besteht, das den vollständigen Abstand bis zur Zellmembran überspannt, wobei das Brückenpeptid an eine Sequenz gebunden ist, die die erforderliche Ankerfunktion ausüben kann und die gegebenenfalls die vollständige oder partielle Transmembran-Domäne und/oder cytoplasmatische Domäne eines Moleküls ist, das ausgewählt ist aus der Gruppe bestehend aus:
(i) einem menschlichen MHC-Klasse-I-Molekül ausgewählt aus einem HLA-A-, HLA-B- oder HLA-C-Molekül;
(ii) einem ko-stimulierenden B7.1-, B7.2- oder CD40-Molekül; und
(iii) einem Signaltransduktionselement, das zur Aktivierung von T-Zellen oder antigenpräsentierenden Zellen fähig ist.

16. DNA-Impfstoff, umfassend ein Polynucleotid nach einem der Ansprüche 1 bis 13 oder einen Expressionsvektor nach Anspruch 14.

17. Zellulärer Impfstoff, der eine antigenpräsentierende Zelle nach Anspruch 15 umfasst.

18. Zellulärer Impfstoff, umfassend antigenpräsentierende Zellen, die ein β₂-Mikroglobulinmolekül exprimieren, das über seinen Carboxy-Terminus an einen Polypeptid-Abschnitt gebunden ist, der die Verankerung des β₂-Mikroglobulinmoleküls an der Zellmembran ermöglicht, wobei der Polypeptidabschnitt am Carboxy-Terminus des β₂-Mikroglobulins aus einem Brückenpeptid besteht, das den vollständigen Abstand bis zur Zellmembran überspannt, wobei das Brückenpeptid aus etwa 10-15 Aminosäuren innerhalb der membranproximalen Sequenz des HLA-Klasse-I-Moleküls der schweren Kette an eine Sequenz gebunden ist, die die erforderliche Ankerfunktion ausüben kann, und die gegebenenfalls die vollständige oder partielle Transmembran-Domäne und/oder cytoplasmatische Domäne eines Moleküls ist, das ausgewählt ist aus der Gruppe bestehend aus:
(i) einem menschlichen MHC-Klasse-I-Molekül ausgewählt aus einem HLA-A-, HLA-B- oder HLA-C-Molekül;
(ii) einem ko-stimulierenden B7.1-, B7.2- oder CD40-Molekül; und
(iii) einem Signaltransduktionselement, das zur Aktivierung von T-Zellen oder antigenpräsentierenden Zellen fähig ist, wobei die Zellen mit mindestens einem antigenen Peptid, das aus mindestens einem Tumor-assoziierten Antigen stammt, gepulst wurden.

19. Antigenpräsentierende Zellen, die ein β₂-Mikroglobulinmolekül exprimieren, das über seinen Carboxy-Terminus an einen Polypeptidabschnitt gebunden ist, der die Verankerung des β₂-Mikroglobulinmoleküls an der Zellmembran ermöglicht, wobei der Polypeptidabschnitt am Carboxy-Terminus des β₂-Mikroglobulins aus einem Brückenpeptid von etwa 10-15 Aminosäuren innerhalb der membranproximalen Sequenz eines HLA-Klasse-I-Moleküls der schweren Kette besteht, das den vollständigen Abstand bis zur Zellmembran überspannt, wobei das Brückenpeptid an eine Sequenz gebunden ist, die die erforderliche Ankerfunktion ausüben kann und die gegebenenfalls die vollständige und/oder partielle Transmembran-Domäne und/oder cytoplasmatische Domäne eines Moleküls ist, das ausgewählt ist aus der Gruppe bestehend aus:
(i) einem menschlichen MHC-Klasse-I-Molekül ausgewählt aus einem HLA-A-, HLA-B- oder HLA-C-Molekül;
(ii) einem ko-stimulierenden B7.1-, B7.2- oder CD40-Molekül; und
(iii) einem Signaltransduktionselement, das zur Aktivierung von T-Zellen oder antigenpräsentierenden Zellen fähig ist, wobei die Zellen mit mindestens einem antigenen Peptid, das aus mindestens einem Tumor-assoziierten Antigen stammt, gepulst wurden,
zur Verwendung in der Vorbeugung oder Behandlung von Krebs.

20. Zellulärer Impfstoff nach Anspruch 17 oder 18, wobei die antigenpräsentierende Zelle ausgewählt ist aus der Gruppe bestehend aus einer dendritischen Zelle, einem Makrophagen, einer B-Zelle und einem Fibroblasten, wobei die antigenpräsentierende Zelle mindestens ein antigenes Peptid präsentiert, das von mindestens einem Tumor-assoziierten Antigen stammt.

21. Antigenpräsentierende Zellen nach Anspruch 19, wobei die antigenpräsentierenden Zellen ausgewählt sind aus der Gruppe bestehend aus dendritischen Zellen, Makrophagen, B-Zellen und Fibroblasten, wobei die antigenpräsentierenden Zellen mindestens ein antigenes Peptid präsentieren, das von mindestens einem Tumor-assoziierten Antigen stammt.

22. Arzneimittel, das als Wirkstoff mindestens ein Polynucleotid nach einem der Ansprüche 1 bis 13 oder einen Expressionsvektor nach Anspruch 14 oder eine antigenpräsentierende Zelle nach Anspruch 15 und einen pharmazeutisch verträglichen Träger umfasst.

23. Expressionsvektor nach Anspruch 14 oder antigenpräsentierende Zelle nach Anspruch 15 zur Verwendung in der Vorbeugung oder Behandlung von Krebs.

## Revendications

1. Polynucléotide comprenant une séquence codant pour un polypeptide qui est capable de présenter un taux élevé de peptides antigènes sur des cellules présentatrices d'antigène, dans lequel le polypeptide comprend une molécule de β₂-microglobuline qui est liée par l'intermédiaire de sa terminaison carboxyle à un allongement polypeptidique permettant l'ancrage de la molécule de β₂-microglobuline à la membrane cellulaire, et par l'intermédiaire de sa terminaison amine à au moins un peptide antigène comprenant un épitope du CMH de classe I qui est un antigène associé à une tumeur (TAA), dans lequel ledit allongement polypeptidique au niveau de la terminaison carboxyle de la β₂-microglobuline se compose d'un peptide pont d'environ 10 à 15 acides aminés dans la séquence proche de la membrane d'une molécule HLA de classe I à chaîne lourde, qui couvre toute la distance jusqu'à la membrane cellulaire, ledit peptide pont étant lié à une séquence qui peut exercer la fonction d'ancrage souhaitée, étant le domaine cytoplasmique et/ou transmembranaire total ou partiel d'une molécule choisie dans le groupe constitué par :
(i) une molécule du CMH de classe I humaine choisie parmi une molécule HLA-A, HLA-B ou HLA-C ;
(ii) une molécule costimulatrice B7.1, B7.2 ou CD40 ; et
(iii) un élément de transduction du signal capable d'activer les lymphocytes T ou les cellules présentatrices d'antigène.

2. Polynucléotide selon la revendication 1, dans lequel ledit peptide pont est le peptide ayant la séquence SEQ ID NO : 1.

3. Polynucléotide selon la revendication 1, dans lequel ledit peptide pont est lié aux domaines transmembranaires et cytoplasmiques de la molécule HLA-A2 à chaîne lourde du CMH de classe I ayant la séquence SEQ ID NO : 2.

4. Polynucléotide selon la revendication 1, dans lequel ledit élément de transduction capable d'activer les lymphocytes T est choisi parmi :
(i) un composant du récepteur CD3 des lymphocytes T tels que le polypeptide zêta
(ζ) ou êta (η), comprenant facultativement des régions transmembranaires et cytoplasmiques du polypeptide CD3 ζ humain ;
(ii) un polypeptide du récepteur des lymphocytes B ; et
(iii) un polypeptide du récepteur Fc.

5. Polynucléotide selon la revendication 1 ou 2, dans lequel ledit peptide pont est lié par l'intermédiaire de sa terminaison carboxyle à une séquence d'ancrage GPI telle que le peptide ayant la séquence SEQ ID NO : 3.

6. Polynucléotide selon l'une quelconque des revendications 1 à 5, dans lequel ledit ou lesdits peptides antigènes comprenant un épitope du CMH de classe I sont liés à la terminaison amine de la β₂-microglobuline par l'intermédiaire d'un lieur peptidique, et ledit ou lesdits peptides antigènes peuvent être au moins un déterminant antigène d'un antigène unique ou au moins un déterminant antigène de chacun d'au moins deux antigènes différents.

7. Polynucléotide selon la revendication 6, dans lequel ledit antigène est un antigène associé à une tumeur (TAA) tel que l'alpha-fétoprotéine, BA-46/lactadhérine, BAGE, la protéine de fusion BCR-ABL, la bêta-caténine, CASP-8, CDK4, CEA, CRIPTO-1, le facteur d'élongation 2, la protéine de fusion ETV6-AML1, G250, GAGE, gp100, HER-2/neu, la carboxylestérase intestinale, KIAA0205, MAGE, MART-1/Melan-A, MUC-1, N-ras, p53, PAP, PSA, PSMA, la télomérase, TRP-1/gp75, TRP-2, la tyrosinase, et l'uroplakine Ia, Ib, II et III.

8. Polynucléotide selon la revendication 7, dans lequel ledit peptide antigène est choisi dans le groupe constitué par :
(i) le peptide d'alpha-fétoprotéine ayant la séquence SEQ ID NO : 4 ;
(ii) le peptide de BAGE-1 ayant la séquence SEQ ID NO : 5 ;
(iii) le peptide de la protéine de fusion BCR-ABL ayant la séquence SEQ ID NO:6 ;
(iv) le peptide de la bêta-caténine ayant la séquence SEQ ID NO : 7 ;
(v) le peptide de CDK4 ayant la séquence SEQ ID NO : 8 ;
(vi) le peptide de CEA ayant la séquence SEQ ID NO : 9 ;
(vii) le peptide du facteur d'élongation 2 ayant la séquence SEQ ID NO : 10 ;
(viii) le peptide de la protéine de fusion ETV6-AML1 ayant la séquence SEQ ID NO : 11 ;
(ix) le peptide de G250 ayant la séquence SEQ ID NO : 12 ;
(x) le peptide de GAGE-1,2,8 ayant la séquence SEQ ID NO : 13 ;
(xi) les peptides de gp100 ayant les séquences SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25 et SEQ ID NO : 26 ;
(xii) le peptide d'HER-2/neu ayant la séquence SEQ ID NO : 27 ;
(xiii) le peptide de la carboxylestérase intestinale ayant la séquence SEQ ID NO : 28 ;
(xiv) le peptide de KIAA0205 ayant la séquence SEQ ID NO : 29 ;
(xv) les peptides de MAGE-1 ayant les séquences SEQ ID NO : 30 et SEQ ID NO:31 ;
(xvi) les peptides de MAGE-3 ayant les séquences SEQ ID NO : 32 et SEQ ID NO:33 ;
(xvii) le peptide de MART-1/Melan-A ayant la séquence SEQ ID NO : 34 ;
(xviii) le peptide de MUC-1 ayant la séquence SEQ ID NO : 35 ;
(xix) le peptide de N-ras ayant la séquence SEQ ID NO : 36 ;
(xx) le peptide de p53 ayant la séquence SEQ ID NO : 37 ;
(xxi) les peptides de PSA ayant les séquences SEQ ID NO : 38 et SEQ ID NO : 39 ;
(xxii) le peptide de la télomérase ayant la séquence SEQ ID NO : 40 ;
(xxiii) le peptide de TRP-1 ayant la séquence SEQ ID NO : 41 ;
(xxiv) les peptides de TRP-2 ayant les séquences SEQ ID NO : 42, SEQ ID NO : 43 et SEQ ID NO : 44 ;
(xxv) le peptide de TRP2-INT2 ayant la séquence SEQ ID NO : 45 ; et
(xxvi) le peptide de tyrosinase ayant la séquence SEQ ID NO : 46.

9. Polynucléotide selon la revendication 7 ou 8, dans lequel ledit ou lesdits peptides antigènes sont au moins un déterminant antigène d'un antigène associé à une tumeur unique.

10. Polynucléotide selon la revendication 9, dans lequel ledit ou lesdits peptides antigènes sont au moins un peptide de liaison à HLA-A2 et au moins un peptide de liaison à HLA-A3 dérivés de l'antigène gp100 associé au mélanome.

11. Polynucléotide selon la revendication 10, dans lequel ledit ou lesdits peptides de liaison à HLA-A2 dérivés de gp100 sont choisis dans le groupe constitué par les séquences SEQ ID NO : 14, 15, 16, 17, 18, 19, 20, 21 et 22 ; et ledit ou lesdits peptides de liaison à HLA-A3 dérivés de gp100 sont choisis dans le groupe constitué par les séquences SEQ ID NO : 23, 24, 25 et 26.

12. Polynucléotide selon la revendication 7 ou 8, dans lequel ledit ou lesdits peptides antigènes sont au moins un déterminant antigène de chacun d'au moins deux antigènes associés à une tumeur différents.

13. Polynucléotide selon la revendication 12, dans lequel ledit ou lesdits peptides antigènes sont au moins un peptide de liaison à HLA-A2 dérivé de chacun des antigènes gp100 et Melan-A/MART-1 associés au mélanome ou au moins un peptide de gp100 retreint à HLA-A3 et au moins un peptide de Melan-A/MART-1 restreint à HLA-A2.

14. Vecteur d'expression, de préférence un vecteur viral recombinant, comprenant un polynucléotide selon l'une quelconque des revendications 1 à 13.

15. Cellule présentatrice d'antigène choisie dans le groupe constitué par une cellule dendritique, un macrophage, un lymphocyte B ou un fibroblaste, transfectée avec un polynucléotide comprenant une séquence codant pour un polypeptide comprenant une molécule de β₂-microglobuline qui est liée par l'intermédiaire de sa terminaison carboxyle à un allongement polypeptidique permettant l'ancrage de la molécule de β₂-microglobuline à la membrane cellulaire, et par l'intermédiaire de sa terminaison amine à au moins un peptide antigène comprenant un épitope du CMH de classe I qui est un antigène associé à une tumeur (TAA), dans lesquelles ledit allongement polypeptidique au niveau de la terminaison carboxyle de la β₂-microglobuline se compose d'un peptide pont d'environ 10 à 15 acides aminés dans la séquence proche de la membrane d'une molécule HLA de classe I à chaîne lourde, qui couvre toute la distance jusqu'à la membrane cellulaire, ledit peptide pont étant lié à une séquence qui peut exercer la fonction d'ancrage souhaitée, étant facultativement le domaine cytoplasmique et/ou transmembranaire total ou partiel d'une molécule choisie dans le groupe constitué par :
(i) une molécule du CMH de classe I humaine choisie parmi une molécule HLA-A, HLA-B ou HLA-C ;
(ii) une molécule costimulatrice B7.1, B7.2 ou CD40 ; et
(iii) un élément de transduction du signal capable d'activer les lymphocytes T ou les cellules présentatrices d'antigène.

16. Vaccin à ADN comprenant un polynucléotide selon l'une quelconque des revendications 1 à 13 ou un vecteur d'expression selon la revendication 14.

17. Vaccin cellulaire qui comprend une cellule présentatrice d'antigène selon la revendication 15.

18. Vaccin cellulaire comprenant des cellules présentatrices d'antigène qui expriment une molécule de β₂-microglobuline liée par l'intermédiaire de sa terminaison carboxyle à un allongement polypeptidique permettant l'ancrage de la molécule de β₂-microglobuline à la membrane cellulaire, dans lequel ledit allongement polypeptidique au niveau de la terminaison carboxyle de la β₂-microglobuline se compose d'un peptide pont qui couvre toute la distance jusqu'à la membrane cellulaire, ledit peptide pont d'environ 10 à 15 acides aminés dans lequel la séquence proche de la membrane d'une molécule HLA de classe I à chaîne lourde étant lié à une séquence qui peut exercer la fonction d'ancrage souhaitée, étant facultativement le domaine cytoplasmique et/ou transmembranaire total ou partiel d'une molécule choisie dans le groupe constitué par :
(i) une molécule du CMH de classe I humaine choisie parmi une molécule HLA-A, HLA-B ou HLA-C ;
(ii) une molécule costimulatrice B7.1, B7.2 ou CD40 ; et
(iii) un élément de transduction de signal capable d'activer les lymphocytes T ou les cellules présentatrices d'antigène, dans lequel lesdites cellules ont été pulsées avec au moins un peptide antigène dérivé d'au moins un antigène associé à une tumeur.

19. Cellules présentatrices d'antigène qui expriment une molécule de β₂-microglobuline liée par l'intermédiaire de sa terminaison carboxyle à un allongement polypeptidique permettant l'ancrage de la molécule de β₂-microglobuline à la membrane cellulaire, dans lesquelles ledit allongement polypeptidique au niveau de la terminaison carboxyle de la β₂-microglobuline se compose d'un peptide pont d'environ 10 à 15 acides aminés dans lequel la séquence proche de la membrane d'une molécule HLA de classe I à chaîne lourde, qui couvre toute la distance jusqu'à la membrane cellulaire, ledit peptide pont étant lié à une séquence qui peut exercer la fonction d'ancrage souhaitée, étant facultativement le domaine cytoplasmique et/ou transmembranaire total ou partiel d'une molécule choisie dans le groupe constitué par :
(i) une molécule du CMH de classe I humaine choisie parmi une molécule HLA-A, HLA-B ou HLA-C ;
(ii) une molécule costimulatrice B7.1, B7.2 ou CD40 ; et
(iii) un élément de transduction du signal capable d'activer les lymphocytes T ou les cellules présentatrices d'antigène, dans lequel lesdites cellules ont été pulsées avec au moins un peptide antigène dérivé d'au moins un antigène associé à une tumeur,
pour une utilisation dans la prévention ou le traitement d'un cancer.

20. Vaccin cellulaire selon la revendication 17 ou 18, dans lequel la cellule présentatrice d'antigène est choisie dans le groupe constitué par une cellule dendritique, un macrophage, un lymphocyte B et un fibroblaste, dans lequel ladite cellule présentatrice d'antigène présente au moins un peptide antigène dérivé d'au moins un antigène associé à une tumeur.

21. Cellules présentatrices d'antigène selon la revendication 19, dans lesquelles les cellules présentatrices d'antigène sont choisies dans le groupe constitué par des cellules dendritiques, des macrophages, des lymphocytes B et des fibroblastes, dans lesquelles lesdites cellules présentatrices d'antigène présentent au moins un peptide antigène dérivé d'au moins un antigène associé à une tumeur.

22. Composition pharmaceutique comprenant en tant que principe actif au moins un polynucléotide selon l'une quelconque des revendications 1 à 13 ou un vecteur d'expression selon la revendication 14, ou une cellule présentatrice d'antigène selon la revendication 15, et un excipient pharmaceutiquement acceptable.

23. Vecteur d'expression selon la revendication 14, ou cellule présentatrice d'antigène selon la revendication 15, pour une utilisation dans la prévention ou le traitement d'un cancer.


### SEQUENCE LISTING

<110> GAVISH-GALILEE BIO APPLICATIONS LTD.
   GROSS, Gideon
   MARGALIT, Alon
<120> MEMBRANE-ANCHORED β2 MICROGLOBULIN COVALENTLY LINKED TO MHC CLASS I
   EPITOPES
<130> GAVISH-004 PCT
<150> US 60/388,273
   <151> 2002-06-12
<160> 64
<170> PatentIn version 3.1
<210> 1
   <211> 13
   <212> PRT
   <213> Synthetic sequence
<400>. 1
<210> 2
   <211> 57
   <212> PRT
   <213> Synthetic sequence
<400> 2
   Val Gly Ile Ile Ala Gly Leu Val Leu Phe Gly Ala Val Ile Thr Gly
   1               5                   10                  15
   Ala Val Val Ala Ala Val Met Trp Arg Arg Lys Ser Ser Asp Arg Lys
               20                  25                  30
   Gly Gly Ser Tyr Ser Gln Ala Ala Ser Ser Asp Ser Ala Gln Gly Ser
           35                  40                  45
   Asp Val Ser Leu Thr Ala Cys Lys Val
       50                  55
<210> 3
   <211> 17
   <212> PRT
   <213> Synthetic sequence
<400> 3
   Phe Thr Leu Thr Gly Leu Leu Gly Thr Leu Val Thr Met Gly Leu Leu
   1 5 10 15
   Thr
<210> 4
   <211> 9
   <212> PRT
   <213> Synthetic sequence
<400> 4
   Gly Val Ala Leu Gln Thr Met Lys Gln
   1 5
<210> 5
   <211> 9
   <212> PRT
   <213> Synthetic sequence
<400> 5
   Ala Ala Arg Ala Val Phe Leu Ala Leu
   1 5
<210> 6
   <211> 9
   <212> PRT
   <213> Synthetic sequence
<400> 6
   Ser Ser Lys Ala Leu Gln Arg Pro Val
   1 5
<210> 7
   <211> 9
   <212> PRT
   <213> Synthetic sequence
<400> 7
   Ser Tyr Leu Asp Ser Gly Ile His Phe
   1 5
<210> 8
   <211> 10
   <212> PRT
   <213> Synthetic sequence
<400> 8
   Ala Cys Asp Pro His Ser Gly His Phe Val
   1 5 10
<210> 9
   <211> 9
   <212> PRT
   <213> Synthetic sequence
<400> 9
   Tyr Leu Ser Gly Ala Asn Leu Asn Leu
   1 5
<210> 10
   <211> 9
   <212> PRT
   <213> Synthetic sequence
<400> 10
   Glu Thr Val Ser Glu Gln Ser Asn Val
   1 5
<210> 11
   <211> 9
   <212> PRT
   <213> Synthetic sequence
<400> 11
   Arg Ile Ala Glu Cys Ile Leu Gly Met
   1 5
<210> 12
   <211> 9
   <212> PRT
   <213> Synthetic sequence
<400> 12
   His Leu Ser Thr Ala Phe Ala Arg Val
   1 5
<210> 13
   <211> 8
   <212> PRT
   <213> Synthetic sequence
<400> 13
   Tyr Arg Pro Arg Pro Arg Arg Tyr
   1 5
<210> 14
   <211> 9
   <212> PRT
   <213> Synthetic sequence
<400> 14
   Lys Thr Trp Gly Gln Tyr Trp Gln Val
   1 5
<210> 15
   <211> 9
   <212> PRT
   <213> Artificial sequence
<220>
   <223> The residue in position 1 is Met or Ala
<400> 15
   Met Leu Gly Thr His Thr Met Glu Val
   1 5
<210> 16
   <211> 9
   <212> PRT
   <213> Synthetic sequence
<400> 16
   Ile Thr Asp Gln Val Pro Phe Ser Val
   1 5
<210> 17
   <211> 9
   <212> PRT
   <213> Synthetic sequence
<400> 17
   Tyr Leu Glu Pro Gly Pro Val Thr Ala
   1 5
<210> 18
   <211> 10
   <212> PRT
   <213> Synthetic sequence
<400> 18
   Leu Leu Asp Gly Thr Ala Thr Leu Arg Leu
   1 5 10
<210> 19
   <211> 10
   <212> PRT
   <213> Synthetic sequence
<400> 19
   Val Leu Tyr Arg Tyr Gly Ser Phe Ser Val
   1 5 10
<210> 20
   <211> 10
   <212> PRT
   <213> Synthetic sequence
<400> 20
   Ser Leu Ala Asp Thr Asn Ser Leu Ala Val
   1 5 10
<210> 21
   <211> 9
   <212> PRT
   <213> Synthetic sequence
<400> 21
   Arg Leu Met Lys Gln Asp Phe Ser Val
   1 5
<210> 22
   <211> 9
   <212> PRT
   <213> Synthetic sequence
<400> 22
   Arg Leu Pro Arg Ile Phe Cys Ser Cys
   1 5
<210> 23
   <211> 9
   <212> PRT
   <213> Synthetic sequence
<400> 23
   Leu Ile Tyr Arg Arg Arg Leu Met Lys
   1 5
<210> 24
   <211> 9
   <212> PRT
   <213> Synthetic sequence
<400> 24
   Ala Leu Leu Ala Val Gly Ala Thr Lys
   1 5
<210> 25
   <211> 10
   <212> PRT
   <213> Synthetic sequence
<400> 25
   Ile Ala Leu Asn Phe Pro Gly Ser Gln Lys
   1 5 10
<210> 26
   <211> 9
   <212> PRT
   <213> Synthetic sequence
<400> 26
   Ala Leu Asn Phe Pro Gly Ser Gln Lys
   1 5
<210> 27
   <211> 9
   <212> PRT
   <213> Synthetic sequence
<400> 27
   Lys Ile Phe Gly Ser Leu Ala Phe Leu
   1 5
<210> 28
   <211> 9
   <212> PRT
   <213> Synthetic sequence
<400> 28
   Ser Pro Arg Trp Trp Pro Thr Cys Leu
   1 5
<210> 29
   <211> 9
   <212> PRT
   <213> Synthetic sequence
<400> 29
   Ala Glu Pro Ile Asn Ile Gln Thr Trp
   1 5
<210> 30
   <211> 9
   <212> PRT
   <213> Synthetic sequence
<400> 30
   Glu Ala Asp Pro Thr Gly His Ser Tyr
   1 5
<210> 31
   <211> 9
   <212> PRT
   <213> Synthetic sequence
<400> 31
   Ser Leu Phe Arg Ala Val Ile Thr Lys
   1 5
<210> 32
   <211> 9
   <212> PRT
   <213> Synthetic sequence
<400> 32
   Glu Val Asp Pro Ile Gly His Leu Tyr
   1 5
<210> 33
   <211> 9
   <212> PRT
   <213> Synthetic sequence
<400> 33
   Phe Leu Trp Gly Pro Arg Ala Leu Val
   1 5
<210> 34
   <211> 9
   <212> PRT
   <213> Artificial sequence
<220>
   <223> The residue in position 1 is Ala or Glu
<400> 34
   Ala Ala Gly Ile Gly Ile Leu Thr Val
   1 5
<210> 35
   <211> 9
   <212> PRT
   <213> Synthetic sequence
<400> 35
   Ser Thr Ala Pro Pro Val His Asn Val
   1 5
<210> 36
   <211> 10
   <212> PRT
   <213> Synthetic sequence
<400> 36
   Ile Leu Asp Thr Ala Gly Arg Glu Glu Tyr
   1 5 10
<210> 37
   <211> 9
   <212> PRT
   <213> Synthetic sequence
<400> 37
   Leu Leu Gly Arg Asn Ser Phe Glu Val
   1 5
<210> 38
   <211> 10
   <212> PRT
   <213> Synthetic sequence
<400> 38
   Phe Leu Thr Pro Lys Lys Leu Gln Cys Val
   1 5 10
<210> 39
   <211> 10
   <212> PRT
   <213> Synthetic sequence
<400> 39
   Val Ile Ser Asn Asp Val Cys Ala Gln Val
   1 5 10
<210> 40
   <211> 9
   <212> PRT
   <213> Synthetic sequence
<400> 40
   Ile Leu Ala Lys Phe Leu His Trp Leu
   1 5
<210> 41
   <211> 9
   <212> PRT
   <213> Synthetic sequence
<400> 41
   Met Ser Leu Gln Arg Gln Phe Leu Arg
   1 5
<210> 42
   <211> 9
   <212> PRT
   <213> Synthetic sequence
<400> 42
   Leu Leu Gly Pro Gly Arg Pro Tyr Arg
   1               5
<210> 43
   <211> 9
   <212> PRT
   <213> Synthetic sequence
<400> 43
   Ser Val Tyr Asp Phe Phe Val Trp Leu
   1               5
<210> 44
   <211> 9
   <212> PRT
   <213> Synthetic sequence
<400> 44
   Thr Leu Asp Ser Gln Val Met Ser Leu
   1               5
<210> 45
   <211> 10
   <212> PRT
   <213> Synthetic sequence
<400> 45
   Glu Val Ile Ser Cys Lys Leu Ile Lys Arg
   1               5                   10
<210> 46
   <211> 9
   <212> PRT
   <213> Synthetic sequence
<400> 46
   Lys Cys Asp Ile Cys Thr Asp Glu Tyr
   1               5
<210> 47
   <211> 10
   <212> PRT
   <213> Synthetic sequence
<400> 47
   Arg Gly Pro Gly Arg Ala Phe Val Thr Ile
   1 5 10
<210> 48
   <211> 8
   <212> PRT
   <213> Synthetic sequence
<400> 48
   Ser Ile Ile Asn Phe Glu Lys Leu
   1 5
<210> 49
   <211> 225
   <212> DNA
   <213> Synthetic sequence
<400> 49
   gagccctcga gctccactgt ctccaacatg gcgaccgttg ctgttctggt tgtccttgga 60
   gctgcaatag tcactggagc tgtggtggct tttgtgatga agatgagaag gagaaacaca 120
   ggtggaaaag gaggggacta tgctctggct ccaggctccc agacctctga tctgtctctc 180
   ccagattgta aagtgatggt tcatgaccct cattctctag cgtga 225
<210> 50
   <211> 13
   <212> PRT
   <213> Synthetic sequence
<400> 50
   Leu Arg Trp Glu Pro Ser Ser Ser Thr Val Ser Asn Met
   1 5 10
<210> 51
   <211> 39
   <212> DNA
   <213> Synthetic sequence
<400> 51
   ctgagatggg agccctcgag ctccactgtc tccaacatg 39
<210> 52
   <211> 30
   <212> DNA
   <213> Synthetic sequence
<400> 52
   ccctcgagct ccactgtctc caacatggcg 30
<210> 53
   <211> 29
   <212> DNA
   <213> Synthetic sequence
<400> 53
   cgcgcggccg caagtccact ccaggcagc 29
<210> 54
   <211> 33
   <212> DNA
   <213> Synthetic sequence
<400> 54
   gcgtctagag cttcagtcgt cagcatggct cgc 33
<210> 55
   <211> 60
   <212> DNA
   <213> Synthetic sequence
<400> 55
   ctgaccggct tgtatgctgt gtatgacttt tttgtgtggc tcggaggtgg cggatccgcg 60
<210> 56
   <211> 60
   <212> DNA
   <213> Synthetic sequence
<400> 56
   cgcggatccg ccacctccga gccacacaaa aaagtcatac acagcataca agccggtcag 60
<210> 57
   <211> 60
   <212> DNA
   <213> Synthetic sequence
<400> 57
   ctgaccggct tgtatgctag tataatcaac tttgaaaaac tgggaggtgg cggatccgcg 60
<210> 58
   <211> 60
   <212> DNA
   <213> Synthetic sequence
<400> 58
   cgcggatccg ccacctccca gtttttcaaa gttgattata ctagcataca agccggtcag 60
<210> 59
   <211> 348
   <212> DNA
   <213> Synthetic sequence
<400> 59
   ggatccggag gtggttctgg tggaggttcg atccagaaaa cccctcaaat tcaagtatac 60
   tcacgccacc caccggagaa tgggaagccg aacatactga actgctacgt aacacagttc 120
   cacccgcctc acattgaaat ccaaatgctg aagaacggga aaaaaattcc taaagtagag 180
   atgtcagata tgtccttcag caaggactgg tctttctata tcctggctca cactgaattc 240
   acccccactg agactgatac atacgcctgc agagttaagc atgacagtat ggccgagccc 300
   aagaccgtct actgggatcg agacatgctg agatgggagc cctcgagc 348
<210> 60
   <211> 55
   <212> DNA
   <213> Synthetic sequence
<400> 60
   gcgggatccg gaggtggttc tggtggaggt tcgatccaga aaacccctca aattc 55
<210> 61
   <211> 45
   <212> DNA
   <213> Synthetic sequence
<400> 61
   gcggctcgag ggctcccatc tcagcatgtc tcgatcccag tagac 45
<210> 62
   <211> 291
   <212> DNA
   <213> Synthetic sequence
<400> 62
   gccctgctgg tcattcctgt cgtgatgggc atcctcatca ccattttcgg ggtgtttctc 60
   tatatcaaaa aggtggtcaa gaaaccaaag gataatgaga tgttaccccc tgcggctcga 120
   cggcaagatc cccaggagat ggaagattat cccggtcata acaccgctgc tccagtgcag 180
   gagacactgc acgggtgtca gcctgtcaca caggaggatg gtaaagagag tcgcatctca 240
   gtgcaggagc ggcaggtgac agacagcata gccttgaggc ccctggtctg a 291
<210> 63
   <211> 46
   <212> DNA
   <213> Synthetic sequence
<400> 63
   ccctcgagct ccactgtctc caacatggcc ctgctggtca ttcctg 46
<210> 64
   <211> 29
   <212> DNA
   <213> Synthetic sequence
<400> 64
   cgcgcggccg cggtcagcaa gcagccatc 29